# EUROPEAN PATENT APPLICATION

(11) **EP 1 076 099 A2**
(43) Date of publication of application: **14.02.2001**
(21) Application number: 00306563.8
(22) Date of filing: 02.08.2000
(51) Int. Cl.: C12Q 1/68

(54) **Kit for diagnosis of tubercle bacilli**

(30) Priority: 03.08.1999 JP 22035799
(71) Applicant: NISSHINBO INDUSTRIES, INC., Chuo-ku, Tokyo (JP); System Research Incorporation, Tokyo (JP)
(72) Inventor: Suzuki, Yasuhiko, Yamatokoriyama-shi, Nara (JP); Nishida, Michio, Chigasaki-shi, Kanagawa (JP); Takenishi, Soichiro, c/o Nisshinbo Industries,Inc., Chiba-shi, Chiba (JP)
(74) Representative: Tombling, Adrian George

(57) **Abstract**

By using a kit for diagnosis of tubercle bacilli containing a substrate, on which immobilized through a covalent bond is an oligonucleotide which is derived from a gene on tubercle bacilli genome that is responsible for drug resistance for one or more drugs employed in tuberculosis treatment and synthesized based on nucleotide sequences obtained from a wild-type tubercle bacilli susceptible to the drug or drugs and a mutant type tubercle bacilli resistant to at least one of the drugs, differentiation of drug resistance of tubercle bacilli or determination of infection with tubercle bacilli is performed by hybridization of the oligonucleotide with a nucleic acid derived from a microorganism to be tested.

## Description

### BACKGROUND OF THE INVENTION

### Field of the Invention

The present invention relates to a kit for diagnosing tubercle bacilli, more precisely, to a kit which is used to obtain information on drug resistance for clinical examination of a tuberculosis patient, in particular, for selection of an appropriate therapeutic agent, and which enables prompt differentiation of drug resistance of tubercle bacilli and determination of infection with tubercle bacilli. Further, the present invention also relates to a kit which makes it possible to simultaneously perform determination of infection and classification of atypical acid-fast bacilli, which belong to the genus *Mycobacterium* like tubercle bacilli. Furthermore, the present invention relates to an oligonucleotide and a nucleic acid probe used for the kit, as well as processes for producing them.

### Related Art

### 1. Drug Resistance Acquisition Mechanism in Tubercle Bacilli Infection and Importance of Prior Information on Drug Resistance

It is known that drug resistance of tubercle bacilli (*Mycobacterium* tuberculosis) is induced by spontaneous random mutations. The frequency depends on the drug, and, for example, the incidences of resistance to major drugs such as rifampicin (RFP), streptomycin (SM), ethambutol (EB), isoniazid(INH) and kanamycin (KM) are in the order of 1 in 10⁷, 1 in 10⁶, 1 in 10⁴, 1 in 10⁶ and 1 in 10⁶, respectively (1, 15). Although there is no data on the incidence of resistance to pyrazinamide, of which use was approved in 1996, there have already been reports on the point mutations inducing resistant organisms (1, 15).

It is thought that the bacterial population of tubercle bacilli in a tubercular cavitary lesion is usually in the order of 10⁸ organisms. If INH is administered to a susceptible patient carrying 10⁹ organisms as a single-drug treatment regimen, 10³ organisms survive as INH-resistant bacilli on average. Although the patient appears to have recovered by this treatment, growth of surviving INH-resistant bacilli leads to another onset of the disease. If RFP is used for re-treatment of this patient, these bacilli acquire resistance to RFP as well by the similar mechanism. Thus, these tubercle bacilli organisms eventually acquire resistance to both drugs, INH and RFP. Repetition of such treatment results in intractable multidrug-resistant tubercle bacilli.

In response to this problem, two-drug combined therapy using INH and RFP and three-drug combined therapy with further addition of another drug have become the mainstream tuberculosis treatment. If INH is administered, not as a single-drug regimen but in combination with another drug such as RFP, to a patients with tubercle bacilli susceptible to drugs from the stage of initial treatment, the incidence of resistant organisms is reduced to an almost negligible level. That is, in theory, the incidence of resistant bacilli is 10⁻⁶ x 10⁻⁷ = 10⁻¹³, that is 1 in 10¹³. It can be considered that almost no resistant bacilli appear in a usual tuberculosis patient. In principle, however, it can be considered that, if tubercle bacilli carried by a patient already has resistance to one of the two drugs, the same result as from single-drug regimen is induced and that resistance to the other drug is acquired with high probability.

For these reasons, if information on drug resistance is obtained prior to treatment, drugs to be administered can be selected excluding those to which resistance has been acquired, and thus the possibility of treatment failures can be made extremely low.
Further, the incidence of new resistant organisms can be reduced to an almost negligible level.

### 2. Genes Associated with Drug Resistance of tubercle bacilli

Researches on the drug resistance acquisition mechanism of tubercle bacilli, which have long been conducted, have revealed that the resistance is not propagated by R-plasmid, transposon or the like as seen in other bacteria. Therefore, resistance is not acquired to many drugs at once, but a mutation is individually inserted into a site on the tubercle bacilli genome DNA on which each drug acts.

It has been found that a point mutation, which is not seen in wild-type tubercle bacilli (tubercle bacilli having no resistance to RFP), is present in a small region on the gene encoding the β-subunit of the RNA polymerase (rpoB) of tubercle bacilli having resistance to rifampicin (RFP) (1, 8, 9).

Mutations on the gene encoding 16S ribosomal RNA (rrs) and the gene encoding ribosomal S12 protein L (rpsL) are associated with the streptomycin (SM) resistance. A region, which is different from that associated with the streptomycin resistance, on the rrs gene is associated with the kanamycin (KM) resistance (1, 10, 11, 16, 22).

It has been revealed that a mutation on the emb operon is associated with the ethambutol (EB) resistance. Mutations on the catalase-peroxidase gene (KatG) as well as the inhA and aphC/oxyR genes are associated with the isoniazid (INH) resistance (1, 8, 12, 13, 14).

It has been found in drug-resistant tubercle bacilli that point mutation that is not seen in wild-type tubercle bacilli is detected in a specific region on a gene associated with resistance and that, on the contrary, drug resistance can be differentiated if point mutation on these genes can be detected.

### 3. Determination of Tuberculosis and Acid-Fast Bacilli Infection and Current Situation of Drug Susceptibility Test

In recent years, remarkable improvement has been seen in bacteriological testing for *Mycobacteria*(tubercle bacillus, *Mycobacterium leprae, Mycobacterium smegmatis* and the like). In general, *Mycobacteria* are classified into three groups; tubercle bacilli, atypical acid-fast mycobacteria and other particular mycobacteria. Biochemical attribute test or nucleic acid amplification is performed to identify atypical acid-fast mycobacteria. Methods for detecting tubercle bacilli are roughly classified into cultivation and nucleic acid amplification. Cultivation and biochemical characterization take 3 to 8 weeks before results can be obtained. Further, the nucleic acid amplification method currently employed is time consuming because a test is performed individually. As a viable drug susceptibility testing method, usually conceived is cultivation.

### 3-1. Cultivation Test

Various improvements have been made to the cultivation method to increase its detection sensitivity or reduce the number of days required for cultivation. Immunochromatography using antibodies against tuberculosis-specific antigen has rapidly been widespread as a convenient differentiation method. As its procedures, a specimen smeared on a slide is inoculated onto a medium without isolating bacteria and cultured, or a bacterial suspension obtained by isolating and culturing bacteria is proliferated on a solid medium or in a liquid medium to investigate presence or absence of colonies and growth of bacteria and then inoculated onto each drug susceptibility test medium to differentiate the resistance. Since tubercle bacilli detection and drug susceptibility testing based on such cultivation take too long, usually 6 to 8 weeks, before the results can be obtained, various modified methods are being developed particularly for tubercle bacilli cultivation. The examples contain the BACTEC system, the MGIT method, the Septi-Check AFB method (all are available from Becton Dickinson), the Bact/ALERT method (Organon Technica) and so on (5, 6, 7). However, even if these modified methods are employed, it takes at least 2 to 3 weeks to differentiate the drug resistance. Considering the mechanism of emergence of resistant bacilli, these methods are far from responding to needs in clinical practice.

### 3-2. Nucleic Acid Amplification Methods

In the nucleic acid amplification methods, probes are used to amplify genes (DNA or RNA) by the PCR method (polymerase chain reaction kit: Roche) or the MTD method similar thereto (using reverse transcriptase and RNA polymerase: Chugai Pharmaceutical) so that presence of the tubercle bacilli gene is checked. They have many advantages as quick test methods because tubercle bacilli can be detected in about 7 hours including the time required for preparation of specimens (about 2 hours for PCR or MTD alone). Although these methods have not been established as methods of investigating drug resistance, they can be employed as a drug resistance test to some extent with technical application.

For example, Telenti et al. sequenced the rpoB gene by using an automatic sequencer and identified the mutation site associated with rifampicin resistance (8). However, this method is not practical because it requires sequencing of a large number of genes and a costly apparatus. Thus, the PCR-SSCP (PCR-single strand conformational polymorphism) method, the heterodupulex method, the PCR-RFLP (PCR-restriction fragment length polymorphism) method, the molecular beacon method and so forth have been developed as a practical gene amplification method.

The PCR-SSCP method utilizes changes in electrophoresis mobility of DNA or RNA according to the high-order structure thereof, which is dependent on the nucleotide sequences. Nucleic acids of the rpoB gene are amplified (PCR) by using an oligo primer, thermally denatured, and subjected to electrophoresis, and then emergence of mutations is judged by detecting differences in the obtained DNA mobility (17).

In the PCR-RFLP method, the rpoB gene site is amplified by PCR. The fragment cleaved by using a restriction enzyme is separated by electrophoresis. Presence or absence of resistance is judged by classifying obtained electrophoresis patterns, (18).

In the heterodupulex method, as in the PCR-SSCP method, after PCR products are thermally denatured and annealed with control DNA fragments, differences in mobility are investigated on the gel electrophoresis.

The molecular beacon method uses an oligonucleotide having a stem-loop structure where a fluorescent substance and a quencher are bound to 5' end and 3' end, respectively. This method utilizes the phenomenon that, if double strand is formed with the PCR product of which target is the oligonucleotide, the physical distance between the fluorescent substance and the quencher is increased (19).

However, these methods have problems such that the length of DNA fragments to be tested is restricted, the mutation site associated with drug resistance requires a site cleaved by a restriction enzyme or a number of experiments need to be performed in parallel to detect a mutation pattern. Although there have been many efforts to detect mutations and research and development of quick test methods have been undertaken, no effective and quick drug resistance test method has been reported so far.

### 3-3. DNA Microarray Technique

A DNA microarray comprises oligonucleotide probes arranged on a silicon chip at high density in an array. The technique developed by Affymax has been introduced as representative one (27, 28). In this technique, the array is constituted by arranging a number of nucleotide sequence probes (consisting of sense or anti-sense) having 1 or 2 interrogation sites at arbitrary locations with respect to reference DNA sequences, to which interrogation sites different nucleotides are used. Since the probes proceed on the reference sequences with overlapping, the number of the probes depends on the length of the sequence to be tested. In principle, a sample DNA to be tested is sequenced in comparison with the reference nucleotide sequence by applying the sample DNA to the probe on a chip and judging their fusion.

It is indicated in the patent application of Affymetrix Inc. that this method can be applied in drug resistance testing of tubercle bacilli. However, any working examples are not described clearly. Although the DNA microarray technique is excellent in determining a large number of unknown sequences, its applicability to drug resistance differentiation, which requires instant diagnosis results, is still in doubt. There also remain technological and economical problems such as chip preparation cost, formation of a precise array on a silicon chip and high-resolution device for reading results (scanner).

### 3-4. Line probe assay applying PCR method

Recently, a line probe assay (INNO-LiPA Rif.TB kit: Innogenetics, Belgium) was developed for the purpose of detection of rifampicin resistance (26, not introduced in Japan). Four fragments from nucleotide sequences on the rpoB gene containing the site of point mutation occurring at a high rate and 5 fragments obtained from nucleotide sequences on the rpoB gene of a rifampicin- susceptible strain are adsorbed on a filter in stripes as probes for detection. Tubercle bacilli DNA amplified by PCR is applied to these probes and their fusion is detected by gel electrophoresis. If bands are observed on the 4 probes containing mutations or bands are not detected on the probes derived from wild-type tubercle bacilli, rifampicin resistance is determined. It is reported that the time required for testing is 96 hours or less after receipt of a specimen. Although this method has significantly improve conventional methods, there still remain a few problems to fully meet needs of accuracy and speed of diagnosis in clinical practice.

Since this is a method where oligonucleotides are adsorbed onto a filter, the number of lines positioned in a unit area is limited. For example, if more mutant sequences associated with rifampicin resistance are to be covered, there will be nearly 20 kinds of sequences as for positive sequences alone. Consequently, many kinds of filters must be prepared. In the aforementioned INNO-LiPA Rif.TB kit, in order to avoid this problem, 5 sequences isolated from different sites on the genome derived from wild-type tubercle bacilli are used and those that are not completely fused are determined as resistant for convenience. However, it is possible that false resistance information is obtained because it is not necessarily evident that the mutation is associated with resistance. Size remains as a problem to further expand this kit to use for testing multi-drug resistance. Since a larger amount of sample DNA is theoretically required, it might take more time to obtain DNA from bacteria

### 4. Differentiation of Drug-Resistant Tubercle Bacilli and Measure of Treatment

To respond to drug-resistant bacilli, a rapid drug resistance differentiation method (ideally, information on drug resistance can be obtained prior to treatment) is preferable. Inevitably, development of novel test methods by gene (DNA or RNA) diagnosis is expected.

As described above, many of genes associated with resistance to major anti-tubercular drugs have been elucidated by many researchers including the present inventors' research group. For example, mutations associated with the rifampicin resistance are concentrated in the core region of only 81 nucleotides on the rpoB gene. The mutations in this region account for 95% of all incidences of resistance (8, 9). It has been revealed that the inhA and aphC/oxyR genes are associated with hydrazide resistance besides the katG gene (13, 21). A plurality of regions located separately on the rrs gene and the rpsL gene are associated with streptomycin resistance (10, 11). Regions, different from those associated with the streptomycin resistance, on the rrs gene are associated with the kanamycin resistance (16, 22). Mutations on the emb operon are associated with ethambutol resistance (12, 23). About 70 to 95% of incidences of resistance to each drug can be explained by analyzing data on mutations in specific regions on a gene. Development of quick drug resistance differentiation methods utilizing this information is of extreme importance in taking countermeasures against tuberculosis problems.

If information on drug resistance can be obtained prior to treatment, not only effective prescription can be provided for treatment, but also emergence of resistant bacilli can be prevented, and thus it is possible that the incidence of nosocomial infection and mass infection, which have recently been a serious concern for our community, is dramatically reduced.

The most emphasized in considering response to tuberculosis problems is provision of information on resistance to major drugs prior to treatment of a patient.

Currently available common methods for drug resistance testing are based on cultivation. It requires at least 2 to 3 weeks before colonies of tubercle bacilli are obtained. Further, they need to be inoculated onto a drug susceptibility test medium to check presence or absence of resistance. Although there have been efforts to improve the nucleic acid amplification method and its modified methods, these methods cannot sufficiently respond to needs in clinical practice due to their defects in convenience and accuracy.

The line probe assay as novel technique has various improvements over the conventional methods in these defects. However, the problem is difficulty in adsorbing many probes onto one test kit. Specimens to be applied tend to have low density in a smear state. That is, it is necessary to increase the number of nucleotide sequences having mutations that can be adsorbed on a kit to enable a multidrug resistance testing and increase the detection rate of mutations. Improvement is also required on the system that can test with a small amount of specimens due to good binding conditions at fusion. It is desired that the detection time is to be further reduced.

### SUMMARY OF THE INVENTION

An object of the present invention is to provide a novel method of differentiating drug resistance of tubercle bacilli and a kit used for this method that can solve most of the problems in currently available testing methods and enables quick diagnosis. Another object of the present invention is to provide a kit and a method designed to enable determination of infection with tubercle bacilli and atypical acid-fast mycobacteria simultaneously.

The present invention provides a kit for diagnosis of tubercle bacilli containing a substrate, on which immobilized through a covalent bond is an oligonucleotide which is derived from a gene on tubercle bacilli genome that is responsible for drug resistance for one or more drugs employed in tuberculosis treatment and synthesized based on nucleotide sequences obtained from a wild-type tubercle bacilli susceptible to the drug or drugs and a mutant type tubercle bacilli resistant to at least one of the drugs, wherein differentiation of drug resistance of tubercle bacilli or determination of infection with tubercle bacilli is attained by hybridization of the oligonucleotide with a nucleic acid derived from a microorganism to be tested.

The present invention also provides a kit for identifying infection with a *Mycobacteria* by hybridization with a nucleic acid derived from a bacterium to be tested, which comprises a substrate on which immobilized through a covalent bond is an oligonucleotide synthesized based on nucleotide sequences derived from genes on genome of *Mycobacteria* other than tubercle bacilli.

The present invention also provides an oligonucleotide which is derived from a mutant type gene having a point mutation responsible for the drug resistance contained in a mutant type tubercle bacilli which is resistant to any one of drugs selected from rifampicin, streptomycin, kanamycin, isoniazid and ethambutol, and designed and synthesized so as to have a length of 12-24 nucleotides containing the mutation.

The present invention further provides an oligonucleotide, wherein the oligonucleotide is a nucleotide sequence obtained by extending or shortening a nucleotide sequence from which the aforementioned oligonucleotide is derived as a base sequence at the 5' end and/or the 3' end with or for a gene sequence of a wild-type tubercle bacilli of which position on the tubercle bacilli genome corresponds to the nucleotide sequence, and the mutation contained in the original nucleotide sequences is not substituted.

The present invention also provides an oligonucleotide which is derived from a wild-type gene contained in a wild-type tubercle bacilli which is susceptible to rifampicin, streptomycin, kanamycin, isoniazid and ethambutol, obtained from a gene sequence of which position on the tubercle bacilli genome corresponds to that of a nucleotide sequence of an oligonucleotide contained in a mutant type gene responsible for resistance to the drugs, and designed and synthesized so as to have a length of 12-24 nucleotides.

The oligonucleotides include DNA, RNA and peptide nucleic acid.

The diagnosis kit of the present invention is characterized in that it comprises a combination of the following (a) and (b):
(a) a nucleotide sequence derived from a mutant type gene having a point mutation responsible for the drug resistance contained in a mutant type tubercle bacilli which is resistant to any one of drugs selected from rifampicin, streptomycin, kanamycin, isoniazid and ethambutol and designed to have a nucleotide number of 12-24, of which representative examples are the nucleotide sequences shown as SEQ ID NOS: 1-66; and
(b) a nucleotide sequence derived from a gene of a wild-type tubercle bacilli which is susceptible to rifampicin, streptomycin, kanamycin, isoniazid and ethambutol and designed to have a nucleotide number of 12-24, of which representative examples are the nucleotide sequences shown as SEQ ID NOS: 67-101.

The present invention further provides, in addition to the aforementioned synthetic oligonucleotide, oligonucleotides of the following (c) and (d):
(c) a synthetic oligonucleotide having a sequence highly conserved in tubercle bacilli and atypical acid-fast mycobacteria and derived from a gene region having a nucleotide sequence that is species specific and is not homologous among *Mycobacteria* species, which served as a positive control;
(d) an oligonucleotide having a nucleotide sequence the oligonucleotide of (c) comprising substitution of one or more nucleotides within a range of from one nucleotide to less than 20% of the total number of nucleotides of the nucleotide sequence, which serves as a negative control.

This oligonucleotide is provided as a component of the aforementioned diagnosis kit or provided independently.

The present invention also provides a method for obtaining a labeled nucleic acid probe used for differentiation of drug resistance of tubercle bacilli or determination of infection with tubercle bacilli or atypical acid-fast bacilli and derived from a nucleic acid of a microorganism to be tested.

That is, a nucleic acid probe labeled with a fluorescent substance is provided by extracting DNA from bacterial cells of a microorganism to be tested obtained from the microorganism to be tested separated from sputum or from culture of the microorganism to be tested; and
performing nucleic acid amplification by employing the DNA as a template and using primers labeled with a fluorescent substance or a hapten at the 5' end or 3' end.

The present invention further provides a method for gene amplification with two steps for carrying out effective gene amplification, and primary primers and secondary primers used therefor.

The present invention further provides primary primers used for the aforementioned production of a nucleic acid probe, which comprises:
a pair of oligonucleotides having the nucleotide sequences shown as SEQ ID NOS: 134 and 135 for amplifying the rpoB gene;
a pair of oligonucleotides having the nucleotide sequences shown as SEQ ID NOS: 136 and 137 for amplifying the rrs gene;
a pair of oligonucleotides having the nucleotide sequences shown as SEQ ID NOS: 138 and 139 for amplifying the rpsL gene;
a pair of oligonucleotides having the nucleotide sequences shown as SEQ ID NOS: 140 and 141 for amplifying the ihhA gene;
a pair of oligonucleotides having the nucleotide sequences shown as SEQ ID NOS: 142 and 143 for amplifying the katG gene;
a pair of oligonucleotides having the nucleotide sequences shown as SEQ ID NOS: 144 and 145 for amplifying the embB gene, or any combination thereof.

The present invention further provides a method for differentiating drug resistance, wherein the aforementioned nucleic acid probe is hybridized with an oligonucleotide immobilized on a substrate to identify an oligonucleotide derived from a gene responsible for drug resistance of a mutant type tubercle bacilli having a nucleotide sequence which is completely identical to the nucleic acid probe, and
a method for determining infection with tubercle bacilli or atypical acid-fast bacilli by comparing association property of the nucleic acid probe with respect to the positive control and the negative control.

By applying the present invention to diagnosis methods of tubercle bacilli infection, both of the differentiation of drug resistance and the determination of infection can be simultaneously performed within 6 hours after receiving a test sample from a patient.

According to the present invention, there is provided a kit enabling quick differentiation of drug resistance of tubercle bacilli and determination of infection with tubercle bacilli. By using the kit of the present invention, it is possible to differentiate resistance to multiple drugs. With the differentiation of drug resistance, infection with tubercle bacilli can be simultaneously diagnosed distinguishing from infection with other *Mycobacteria.* The time required for the test is about 6 hours after receiving a test sample from a patient, and thus it is a quick diagnosis method, which is distinctly discriminated from the prior art techniques. Further, since positive data for drug resistance do not contain false positive results, there is not anxiety for losing a therapeutic opportunity with a specific drug. By using the technique of the present invention, it becomes possible to select a drug suitable for treatment of a specific patient. Thus, emergence of tubercle bacilli resistant to multiple drugs can be suppressed, and effective treatment of tuberculosis can be realized.

### BRIEF EXPLANATION OF THE DRAWINGS

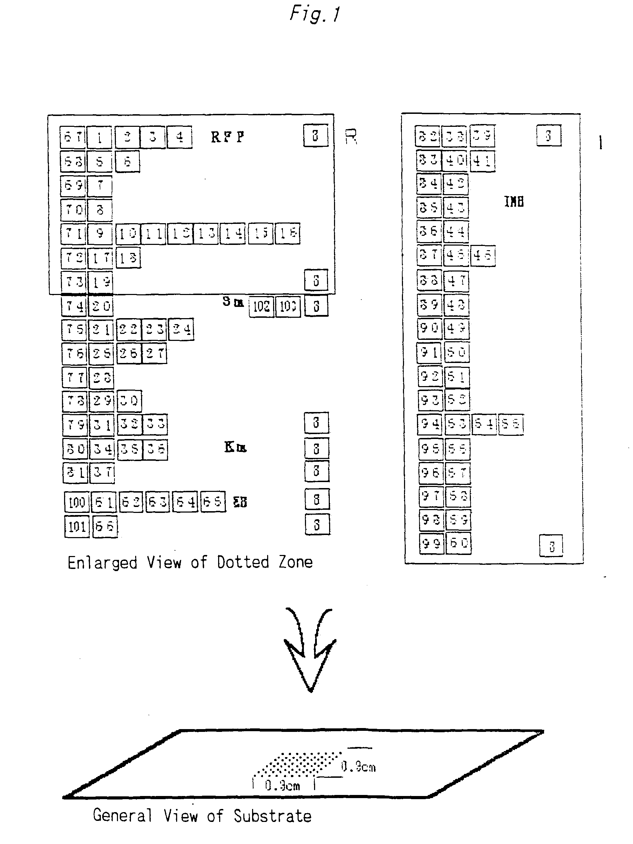

Fig. 1 represents an arrangement of oligonucleotides immobilized on a substrate. The abbreviations in the figure have the following meanings.

RFP: Area where an oligonucleotide for determining rifampicin resistance is immobilized.

Sm: Area where an oligonucleotide for determining streptomycin resistance is immobilized.

Km: Area where an oligonucleotide for determining kanamycin resistance is immobilized.

INH: Area where an oligonucleotide for determining isoniazid resistance is immobilized.

EB: Area where an oligonucleotide for determining ethambutol resistance is immobilized.

B: Area where a biotinylated oligonucleotide is immobilized.

Fig. 2 represents a schematic view of a kit of the present invention and results of detection of rifampicin susceptible tubercle using the kit. The symbols in the figure have the following meanings. The numerals in ● and ○ indicate locations of nucleotide spotting (the same shall apply to the following figures).

●: Spot recognized as positive as determined by pigmentation after chemical color development.

○: Spot recognized as negative as determined by pigmentation after chemical color development.

Fig. 3 represents a schematic view of a kit of the present invention and detection result of rifampicin susceptible tubercle bacilli using the kit (detection result where a signal is obtained at the location of M2).

Fig. 4 represents a schematic view of a kit of the present invention and detection result of rifampicin resistant tubercle bacilli using the kit (detection result where a signal is obtained at the location of M3).

Fig. 5 represents a schematic view of a kit of the present invention and detection result of rifampicin resistant tubercle bacilli using the kit (detection result where a signal is obtained at the location of M4).

Fig. 6 represents a schematic view of a kit of the present invention and detection result of rifampicin resistant tubercle bacilli using the kit (detection result where a signal is obtained at the location of M5).

Fig. 7 represents a schematic view of a kit of the present invention and detection result of rifampicin resistant tubercle bacilli using the kit (detection result where a signal is obtained at the location of M10).

Fig. 8 represents a schematic view of a kit of the present invention and detection result of rifampicin resistant tubercle bacilli using the kit (detection result where a signal is obtained at the location of M11).

Fig. 9 represents a schematic view of a kit of the present invention and detection result of rifampicin resistant tubercle bacilli using the kit (detection result where a signal is obtained at the location of M12).

Fig. 10 represents a schematic view of a kit of the present invention and detection result of rifampicin resistant tubercle bacilli using the kit (detection result where a signal is obtained at the location of M17).

Fig. 11 represents a schematic view of a kit of the present invention and detection result of rifampicin resistant tubercle bacilli using the kit (detection result where a signal is obtained at the location of M18).

Fig. 12 represents a schematic view of a kit of the present invention and detection result of kanamycin susceptible tubercle bacilli using the kit (detection result where signals are obtained at the locations of SEQ ID NOS: 80 and 81).

Fig. 13 represents a schematic view of a kit of the present invention and detection result of kanamycin resistant tubercle bacilli using the kit (detection result where signals are obtained at the locations of SEQ ID NO: 34).

Fig. 14 represents a schematic view of a kit of the present invention and detection result of kanamycin resistant tubercle bacilli using the kit (detection result where signals are obtained at the locations of SEQ ID NOS: 35 and 37).

Fig. 15 represents a schematic view of a kit of the present invention and detection result of kanamycin resistant tubercle bacilli using the kit (detection result where signals are obtained at the locations of SEQ ID NO: 36).

Fig. 16 represents a schematic view of a kit of the present invention and detection result of streptomycin susceptible tubercle bacilli using the kit (detection result where signals are obtained at the locations of SEQ ID NO: 74 and 75).

Fig. 17 represents a schematic view of a kit of the present invention and detection result of streptomycin resistant tubercle bacilli using the kit (detection result where signals are obtained at the locations of SEQ ID NOS: 22).

Fig. 18 represents a schematic view of a kit of the present invention and detection result of streptomycin susceptible tubercle bacilli using the kit (detection result where signals are obtained at the locations of SEQ ID NOS: 76 and 77).

Fig. 19 represents a schematic view of a kit of the present invention and detection result of streptomycin susceptible tubercle bacilli using the kit (detection result where signals are obtained at the locations of SEQ ID NOS: 78 and 79).

Fig. 20 represents a schematic view of a kit of the present invention and detection result of ethambutol susceptible tubercle bacilli using the kit (detection result where signals are obtained at the locations of SEQ ID NOS: 100 and 101).

Fig. 21 represents a schematic view of a kit of the present invention and detection result of isoniazid susceptible tubercle bacilli using the kit (detection result where signals are obtained at the locations of SEQ ID NOS: 82 to 89).

### DESCRIPTION OF PREFERRED EMBODIMENTS

The present invention will be described in detail hereinafter.

In the present invention, susceptibility and resistance to a drug are opposing concepts and the degree of susceptibility or resistance is not limited. If a mutant type tubercle bacilli grows better than a wild-type tubercle bacilli in the presence of a drug at a certain concentration, the mutant type tubercle bacilli is resistant to the drug to which the wild-type tubercle bacilli is susceptible.

In the present invention, drugs are not particularly limited so long as a mutant type tubercle bacilli with susceptibility lower than that of wild-type tubercle bacilli can emerge, but major drugs include rifampicin, streptomycin, kanamycin, isoniazid, ethambutol and so forth. A tubercle bacilli that can grow in the presence of 50 µg/µL of rifampicin, 20 µg/µL of streptomycin, 100 µg/µL of kanamycin, 1 µg/µL of isoniazid or 5 µg/µL of ethambutol can be usually considered to be resistant to these drugs.

The mutant type tubercle bacilli that has drug resistance may be resistant to a single drug or two or more kinds of drugs.

The components and preparation of the kit of the present invention, the method of differentiating drug resistance and the method of identifying infection with tubercle bacilli and atypical acid-fast mycobacteria will be described below.

The components and preparation of the kit of the present invention will be described below.

### <1> Synthetic oligonucleotides used in the present invention

Synthetic oligonucleotides used in the present invention are oligonucleotides derived from a gene on the tubercle bacilli genome responsible for resistance to an antitubercle drug and synthesized based on nucleotide sequences obtained from both wild-type tubercle bacilli that is susceptible to the aforementioned drug and mutant type tubercle bacilli that is resistant any drug. Hereafter, these oligonucleotides are also referred to as "capture oligos". A capture oligo derived from wild-type tubercle bacilli and a capture oligo derived from mutant type tubercle bacilli are also referred to as "wild-type capture oligo" and "mutant type capture oligo", respectively.

Other synthetic oligonucleotides used in the present invention are those derived from a region of a gene having a nucleotide sequence that matches between a drug- susceptible wild-type tubercle bacilli and drug-resistant mutant type tubercle bacilli, but specific to a species and are not homologous among species of *Mycobacteria* (also referred to as "positive control oligos" hereinafter) and those having a nucleotide sequence of any one of the aforementioned positive control oligos containing artificial substitution of one or more nucleotides within a range of less than 20% of the number of nucleotides contained in the nucleotide sequence (also referred to as "negative control oligo" hereinafter). These oligonucleotides are used for determination of infection with tubercle bacilli and atypical acid-fast mycobacteria.

Oligonucleotides used in the present invention will be described below.

### (1) Designing of capture oligos

A tubercle bacilli acquires drug resistance by point mutation on a specific gene. As described above, several such genes responsible for drug resistance have been identified and mutation sites on each gene sequence have also been elucidated (1, 8, 9, 10 to 15). Some of these genes are responsible for resistance to different kinds of drugs such as the rrs gene (responsible for resistance to streptomycin and kanamycin). The essential characteristic of the present invention is that, in summary, presence or absence of drug resistance in tested tubercle bacilli is determined by detecting presence or absence of a mutation on the genes responsible for drug resistance as described above.

In the present invention, the genes responsible for drug resistance include not only those of mutant type tubercle bacilli having drug resistance, but also those of wild-type tubercle bacilli having a nucleotide sequence matching that of the mutant type genes except for the mutation. When locations of the mutant type gene responsible for drug resistance and the wild-type gene match on the tubercle bacilli genome, this relationship is referred with the term "corresponding".

Presence or absence of a mutation in a gene responsible for drug resistance can be determined by attempting to hybridize a nucleic acid fragment probe (referred to as "nucleic acid probe") having a part of a nucleotide sequence on tubercle bacilli chromosome DNA responsible for drug resistance with a wild-type capture oligo and a mutant type capture oligo to see with which capture oligo the nucleotides are hybridized. In other words, it is possible to exactly find to which drug the gene is resistant by the hybridization site on the substrate.

The capture oligos can be prepared as a synthetic oligonucleotide having a nucleotide sequence consisting of 12 to 24 nucleotides containing the mutation point in the nucleotide sequence on a gene responsible for drug resistance. In designing a capture oligo, location of the mutation point in the capture oligo is not particularly limited so long as it can be hybridized with a nucleic acid probe. Usually, however, the location is preferably located at the center of a capture oligo. If the capture oligo is too short, it is difficult to detect hybridization. If it is too long, inhibition of hybridization is not induced by the mutation point. Therefore, the range of 12 to 24 nucleotides is preferred. However, this optimization of the oligo length primarily depends on the characteristics of the sequence (content of specific nucleotides and repetition of the same nucleotide). It has been confirmed in experiments concerning the present invention that short chains that have good binding property can also be used.

Examples of capture oligo nucleotide sequences are listed as SEQ ID NOS: 1 to 101 and in Table 1. Capture oligos of SEQ ID NOS: 1 to 66 are mutant type capture oligos and those of SEQ ID NOS: 67 to 101 are wild-type capture oligos. These capture oligos are designed based on the results of analysis of data on the incidence of drug resistance obtained from published references and the inventors' own tests. The number of nucleotides can be increased up to the level where point mutations occurring for each drug can be widely covered.

Differentiation of resistance to a specific drug can be attained by selecting oligonucleotides from this list and used in a test. The length of capture oligos was generally selected within the range of 14 to 22 nucleotides and lengths within the range of 16 to 17 nucleotides was selected as major lengths in view of hybridization efficiency. As a capture oligo, besides the oligonucleotides shown as SEQ ID NOS: 1 to 66, those having a nucleotide sequence obtained by extending each of the gene sequence of wild-type tubercle bacilli corresponding to the listed nucleotide sequences, at 5' end and/or 3' end, or those having a nucleotide sequence obtained by shortening the nucleotide sequences shown as SEQ ID NOS: 1 to 66 at 5' end or 3' end. One of 5' and 3' ends of nucleotide sequences shown as SEQ ID NOS: 1 to 66 may be extended while the other end is shortened. In either case, however, the length of a capture oligo is within the range of 12 to 24 nucleotides. Nucleotide sequences on wild-type tubercle bacilli genes responsible for drug resistance can be obtained from the GenBank/EMBL/DDBJ database or the like. Accession No. of each gene will be shown below. Extended oligonucleotide sequences can be designed according to these sequences.
rpoB: MTCI376
rrs: MTRRNOP
rpsL: MTV040
inhA: MTCY277
katG: MTKATGCP
embB: MSGY126

To differentiate the resistance to isoniazid, capture oligos can also be designed from the aphC/oxyR gene region. However, since contribution to detection of the drug resistance incidence is low and almost the same detection probability (the same sites are covered) can be achieved by targeting the inhA and katG genes, it is considered that capture oligos derived from the aphC/oxyR gene need not to be used. However, capture oligos derived from the aphC/oxyR gene may also be used. If resistance that cannot be covered by mutations on other genes is found in the aphC/oxyR gene in future, it is preferable that the mutant sequences derived from this gene and the wild-type sequences corresponding thereto are used as capture oligos.

In addition, since some genes have a plurality of mutation sites responsible for drug resistance, a capture oligo specific for each mutation site can be prepared.

When the oligonucleotide sequences designed in the present invention are retrospectively applied in the drug-resistant gene sequence database accumulated from the results of the present inventors' own investigation and reports from references, the expected values of the detection rate were those as shown in Table 2.

Capture oligos designed in the present invention well reflect the results of researches and investigations conducted before the present application. However, if additional information on drug resistance mutation sequences is obtained after the application, novel capture oligos may be designed based on the method described in the present application and they fall within the scope of the present application.

**Table 1**

| | Gene region (Subcode) | Wild-type sequences | | Drug resistance mutation sequences | |
|---|---|---|---|---|---|
| | | Sequence | SEQ ID NO: | Sequence | SEQ ID NO: |
| Rifampicin | RpoB | 7 | 67 to 73 | 19 | 1 to 19 |
| Streptomycin | rrs (530) | 2 | 74 to 75 | 5 | 20 to 24 |
| | rrs (915) | 2 | 76 to 77 | 4 | 25 to 28 |
| | rsL | 2 | 78 to 79 | 5 | 29 to 33 |
| Kanamycin | rrs (1400) | 2 | 80 to 81 | 4 | 34 to 37 |
| Hydrazide | InhA | 2 | 82 to 83 | 4 | 38 to 41 |
| | KatG | 16 | 84 to 99 | 19 | 42 to 60 |
| | aphC/oxyR | 0 | | 0 | |
| Ethambutol | EmbB | 2 | 100 to 101 | 6 | 61 to 66 |
| Total | | 35 | 67 to 101 | 66 | 1 to 66 |
| (Subcode: Site on gene sequence) | | | | | |

**Table 2**

| | Gene region | Detection probability (expectifon value) |
|---|---|---|
| Rifampicin | rpoB | About 95 % |
| Streptomycin | rrs, rpsL | About 80 % |
| Kanamycin | rrs | About 70 % |
| Isoniazid | inhA, katG | About 80 % |
| Ethambutol | embB | About 70 % |

Synthesis of oligonucleotides, and preparation of chromosome DNA, hybridization, PCR and the like to be described later can be performed according to usual methods known to those skilled in the art (refer to Maniatis, T. et al., "Molecular Cloning A Laboratory Manual, Second Edition", Cold Spring Harbor Laboratory Press (1989) and so forth). Oligonucleotides can also be synthesized by using commercially available DNA synthesizers.

When the oligonucleotide has a three-dimensional structure such as a hair-pin structure or a loop structure which blocks hybridization with a nucleic acid to be tested, the three-dimensional structure may be eliminated by replacement of one or more nucleoside which constitutes the oligonucloside with inosine or nucleic acid which does not match with any of nuclosides.

### (2) Designing of control oligos

Whether the patient is infected with tubercle bacilli or atypical acid-fast mycobacteria can be judged by whether the tested bacilli have a gene sequence specific to tubercle bacilli or atypical acid-fast mycobacteria. The determination is done by hybridization with positive control oligos. A negative control oligo is used for the purpose of confirming that hybridization of an oligonucleotide and a nucleic acid probe is specific, that is, there is no signal of false positive hybridization.

Representative models of the nucleotide sequences of the positive control oligo used to identify infection with tubercle bacilli are those shown as SEQ ID NOS: 102 and 160, and representative models of the nucleotide sequences of the negative control oligo are those shown as SEQ ID NOS: 103 and 161. Similarly, there can ben mentioned SEQ ID NOS: 162 and 165 for *Mycobacterium avium* complex, SEQ ID NOS: 164 and 163 for *Mycobacterium kansasii,* SEQ ID NO: 206 and 219 for *Mycobacterium xenopi,* SEQ ID NOS: 207 and 220 for *Mycobacterium ulcerans,* SEQ ID NO: 208 and 221 for *Mycobacterium szulgai,* SEQ ID NOS: 209 and 222 for *Mycobacterium simiae,* SEQ ID NOS: 210 and 223 for *Mycobacterium shimoidei,* SEQ ID NOS: 211 and 224 for *Mycobacterium scrofulaceum,* SEQ ID NO: 212 and 225 for *Mycobacterium nonchromogenicum,* SEQ ID NOS: 213 and 226 for *Mycobacterium marinum,* SEQ ID NO: 214 and 227 for *Mycobacterium malmoense,* SEQ ID NOS: 215 and 228 for *Mycobacterium intracellurare,* SEQ ID NOS: 216 and 229 for *Mycobacterium gordonae,* SEQ ID NOS: 217 and 230 for *Mycobacterium fortuitum,* SEQ ID NOS: 218 and 231 for *Mycobacterium chelonae.* As the negative control oligos, those having substitution of one or more nucleotides, but having 80% or more homology can be mentioned. As for nucleic acid probes obtained from DNA specimens, the range of nucleic acid amplification is selected so as to cover the same regions.

A result of hybridization of a nucleic acid probe and a capture oligo derived from wild-type tubercle bacilli for multi-drug resistance testing may have already provided information enough to judge infection with tubercle bacilli. However, comparison between positive control oligos and negative control oligos further ensures the determination of infection with tubercle bacilli and clearly distinguishes from infection with other *Mycobacteria.*

### <2> Preparation of substrate used in the present invention

Material of a substrate on which oligonucleotides are immobilized is not particularly limited so long as oligonucleotides can be stably immobilized. For example, however, synthetic resin such as glass, polycarbonate and plastic can be mentioned. Shape of the substrate is not particularly limited, but can be a plate or film. A substrate of which surface is uniform and flat is suitable.

To immobilize oligonucleotides onto a substrate, a common methods used for hybridization such as physical adsorption, electrical bond or intermolecular covalent bond can be employed. In examples of the present invention, however, oligonucleotides were immobilized through covalent bonds by using a base material having carbodiimide or isocyanate groups on its surface (Japanese Patent Laid-open (Kokai) No. 8-23975) and irradiating with ultraviolet ray. This is because the immobilization efficiency was significantly increased successfully by irradiating the base material having carbodiimide or isocyanate groups on its surface with ultraviolet ray.

If the amount of oligonucleotides spotted is too small, enough reaction between oligonucleotides and the nucleic acid probes cannot be ensured and this may result in difficulty in determination. On the other hand, spotting at high density incurs technical problems as well as cost. This requires a more accurate and costly detector (representatively a scanner) for fluorescent labelling of nucleic acid probes or determination of presence or absence of hybridization using chemical color development. Therefore, it is preferable that oligonucleotides are immobilizeed in a size of 10 to 1000 µm in diameter on the surface of a substrate. Oligonucleotides can be immobilized by spotting an oligonucleotide solution on the substrate by using, for example, a spotting machine. The oligonucleotide solution is usually spotted in an approximately circular shape.

In addition, a plurality of oligonucleotides are spotted on a single substrate. It is preferable that spots are arranged in a grid pattern. It is advisable that capture oligos containting point mutations and the correponding oligonucleotides derived from wild-type strains are arranged so that comparison would become easy. The number of spots is 1600 or less per cm² in total if the circular spot size is 1000 µm in diameter. If they are spotted in a square pattern, it is preferable to spot in 40 x 40 or less. If the spot size is 10 µm per cm² or less in diameter, the total number is preferably 400 or less. If they are spotted in a square pattern, 20 x 20 or less is preferable. If the width and length are different, the number of spots in the width and length directions can be adjusted depending on the shape.

### <3> Arrangement of oligonucleotides on substrate

Oligonucleotides are preferably arranged on the substrate so that wild-type capture oligos and mutant type capture oligos can be contrasted and resistance or susceptibility of tubercle bacilli to each drug can be easily differentiated. The spotted area on the substrate should be usually within 1 cm².

If the drug is, for example, rifampicin, rifampicin-resistant mutant type capture oligos derived from mutant type tubercle bacilli and wild-type capture oligos corresponding to the murant type capture oligos are positioned so that they can be contrasted. If a plurality of kinds of mutant type capture oligos are used, they are arranged in line. If the test is for a plurality of drugs, such an arrangement as mentioned above can be collectively positioned for each drug. The drug may be one or more kinds of drugs selected from the group consisting of rifampicin, streptomycin, kanamycin, isoniazid and ethambutol. Preferred combinations are a combination of rifampicin and isoniazid and a combination of all the five drugs, rifampicin, streptomycin, kanamycin, isoniazid and ethambutol.

In addition, positive control oligos and negative control oligos are preferably arranged in line so that infection with tuberculosis or atypical acid-fast mycobacteria can be easily determined.

Fig.1 shows an examplary arrangement of oligonucleotides. This example is desinged so that resistance to all of the five drugs, rifampicin, streptomycin, kanamycin, isoniazid and ethambutol can be simulataneously differentiated and infection with tubercle bacilli can be determined. If resistance to only one kind of drug, for example, rifampicin is differentiated, a substrate having the portion enclosed as "R" in Fig.1 can be prepared. If resistance to two kinds of drugs, for example, rifampicin and isoniazid is differentiated, a substrate having the portions enlosed as "R" and "I" in Fig.1 can be prepared.

### <4> Preparation of nucleic acids from test sample and preparation of nucleic acid probes

Nucleic acids can be prepared from test samples by usual methods of preparing nucleic acids from bacteria. For example, DNA can be extracted according to the method introduced by the U.S. Department of Health and Human Services (25) by isolating bacteria from sputum by alkali treatment and disrupting the bacterial genome by the method of Jacobs et al.(24). DNA can be extracted from bacterial cells directly obtained from a culture by using the method of Jacobs et al. These are all standard test methods, but there are many alternative methods. Any of these methods can be employed.

Nucleic acid probes to be used for differentiation of drug resistance or determination of infection are prepared by using the obtained DNA. The nucleic acid probes can be prepared by amplifying nucleic acids using primers designed based on the nucleotide sequences of the capture oligo or the control oligos. DNA is usually used as a nucleic acid probe, but RNA can also be used. Methods of amplifying nucleic acids include, for example, a method of amplifying DNA by polymerase chain reaction (PCR) and a method of amplifying RNA by the in-vitro transcription method.

If a nucleic acid is amplified by PCR, it is preferable that the primary amplification is performed by using primary primers for amplifying a region larger than the target nucleic acid probe to improve specificity of amplification reaction, and then nucleic acid is amplified by using primers for obtaining the target nucleic acid probe and the amplified DNA as a template (two-stage nucleic acid amplification). The primary nucleic acid amplification may be omitted depending on the amount, purity and so forth of the test sample.

The primer used for the final PCR is designed so that the nucleic acid probe should contain a nucleotide sequence complementary to the capture oligo or the control oligo except for the mutation site. So long as hybridization is possible, the nucleic acid probe may be longer or shorter than the capture oligo or the control oligo.

Since some genes have a plurality of mutation sites responsible for drug resistance, a nucleic acid probe for each mutation site can be prepared.

If the primers used for the final PCR are labelled beforehand, a labelled nucleic acid probe can be obtained. The nucleic acid probe can be labelled during or after PCR. As a marker substance, those similar to those for probes used in usual hybridization such as fluorescent substances and haptens can be used. Specifically, for example, fluorescein (FITC), rhodamine, phycoerythrin (PE), Texas Red, cyanin fluorescent dyes and so forth can be mentioned as fluorescent substances. Biotin, digoxigenin (Dig), dinitrophenyl (DNP), fluorescein and the like can be mentioned as the haptens.

Examples of the primers for the primary PCR and the primers for the secondary PCR used in two-stage amplification are mentioned in Table 3. Nucleotide sequences of nucleic acid probes amplified by each set of primers are mentioned in Table 4.

**Table 3**

| | Gene region | Number of probes | Primary primers | Combination of secondary primers |
|---|---|---|---|---|
| Rifampicin | rpoB | 1 | (134, 135) | (106, 107) 146 |
| Streptomyci | rps | 2 | (136, 137) | (108, 109) 147, (110, 111) 148 |
| n | rpsL | 1 | (138, 139) | (112, 113) 149 |
| Kanamycin | rrs | 1 | (136, 137) | (114, 115) 150 |
| Isoniazid | inhA | 1 | (140, 141) | (116, 117) 151 |
| | katG | 7 | (142, 143) | (118, 119) 152, (120, 121) 153 |
| | | | | (122, 123) 154, (124, 125) 155 |
| | | | | (126, 127) 156, (128, 129) 157 |
| | | | | (130, 131) 158 |
| Ethambutol | embB | 1 | (144, 145) | (132, 133) 159 |

Note: "(a, b) c" indicates a combination of sequence numbers (a and b) and a nucleotide sequence of a nucleic acid probe (c) amplified by the combination.

The nucleic acid probe corresponding to the positive control oligo having a nucleotide sequence shown as SEQ ID NO: 102 and the nucleic acid probe corresponding to the negative control oligo having a nucleotide sequence shown as SEQ ID NO: 103 can be prepared by PCR using primers having nucleotide sequences shown as SEQ ID NOS: 104 and 105 and tubercle bacilli genome DNA as a template.

Each positive control oligo and negative control oligo are prepared by using, as a template, tubercle bacilli genome DNA for those of SEQ ID NOS: 160 and 161, *Mycobacterium avium* complex genome DNA for those of SEQ ID NOS: 162 and 165, *Mycobacterium kansasii* genome DNA for those of SEQ ID NOS: 164 and 163, *Mycobacterium xenopi* genome DNA for those of SEQ ID NOS: 206 and 219, *Mycobacterium ulcerans* genome DNA for those of SEQ ID NOS: 207 and 220, *Mycobacterium szulgai* genome DNA for those of SEQ ID NOS: 208 and 221, *Mycobacterium simiae* genome DNA for those of SEQ ID NOS: 209 and 222, *Mycobacterium shimoidei* genome DNA for those of SEQ ID NOS: 210 and 223, *Mycobacterium scrofulaceum* genome DNA for SEQ ID NOS: 211 and 224, *Mycobacterium nonchromogenicum* genome DNA for those of SEQ ID NOS: 212 and 225, *Mycobacterium marinum* genome DNA for those of SEQ ID NOS: 213 and 226, *Mycobacterium malmoense* genome DNA for those of SEQ ID NOS: 214 and 227, *Mycobacterium intracellurare* genome DNA for those of SEQ ID NOS: 215 and 228, *Mycobacterium gordonae* genome DNA for those of SEQ ID NOS: 216 and 229, *Mycobacterium fortuitum* genome DNA for thosse of SEQ ID NOS: 217 and 230 and *Mycobacterium chelonae* genome DNA for those of SEQ ID NOS: 218 and 231. The nucleic acid probes corresponding these positive and negative controls are prepared by using primers shown as SEQ ID NOS: 204 and 205.

Primers for preparing nucleic acid probes can be contained in the tubercle bacilli diagnosis kit together with a substrate on which oligonucleotide is immobilized.

### <5> Hybridization of oligonucleotides and nucleic acid probes on substrate

Hybridization can be performed in the same way as usual nucleic acid hybridization. A specific method will be exemplified below.

A nucleic acid probe is added to a fusion solution consisting of a salt solution such as standard saline citrate (SSC), sodium dodesyl sulfate(SDS), a blocking solution such as bovine serum albumin and an additive for accelerating fusion reaction. If the probe is double-stranded, thermal denaturation is performed. After several pL of the nucleic acid probe solution is added on the substrate and heated for a several hours (usually at 37 to 50°C) to hybridize the oligonucleotide and the nucleic acid probe immobilized on the substrate.

5 x SSC or 3 M tetramethylammonium chloride is added to the substrate and heated (usually at 37 to 50°C) to remove nonspecific hybrids from the substrate and selectively leave only specific hybrids on the substrate.

A fluorescent substance or a hapten introduced into the nucleic acid probe is used to detect hybrids. If a hapten is used, a solution containing a protein that recognizes the hapten or is to be bound thereto and a conjugate (enzyme conjugate) of alkaline phosphatase, horseradish peroxidase or the like is added to the substrate and allowed to react for several tens of minutes at room temperature. Nonspecific adsorption reaction of the enzyme conjugate and the substrate is inhibited by completely coating the regions of the substrate other than the regions on which the oligonucleotide has been immobilized by using a protein such as casein before the binding reaction of the hapten and the enzyme conjugate is performed. This treatment is done by immobilizing the oligonucleotide, then adding a solution of a protein such as casein on the substrate and leaving for several tens of minutes at room temperature.

After the binding reaction of the enzyme conjugate and the hapten in the nucleic acid probe is completed, the enzyme conjugates that are not bound with the hapten are washed down and eliminated with an appropriate buffer solution containing a surfectant so that only the enzyme conjugates bound with the hapten in the nucleic acid probe are left on the substrate.

To visualize hybrids, a compound that becomes insoluble only when the hapten and the enzyme conjugate are present is added so that the insoluble compound generation is amplified by enzymatic reaction and visualized. Compounds used for this purpose include nitroblue tetrazolium chloride (NBT) and 5-bromo-4-chloro-3-indolylphosphoric acid (BCIP) when the enzyme in the enzyme conjugate is alkaline phosphatase. When the enzyme is horseradish peroxidase, 3,3',5,5'-tetramethylbenzidine (TMB) and so forth may be used.

Differentiation of drug resistance based on the obtained hybridization results is performed by examining pigmentation or fluorescent color development at the sites where the capture oligos are immobilized. That is, a site with pigmentation or fluorescent color development indicates the relevant gene. If a positive signal is observed at the site where the wild-type capture oligo is immobilized, any point mutation is not present in the gene responsible for the drug resistance. If a positive signal is observed at a position where the mutant type capture oligo is immobilized, the tested bacterium has a drug resistant mutation sequence.

If a positive signal is observed at a site of positive control oligo of tubercle bacilli or atypical acid-fast mycobacteria, the tested bacterium is a bacterial species corresponding to the positive control. If a positive signal is observed at a site of the negative control oligo of tubercle bacilli or atypical acid-fast mycobacteria, hybridization was not performed under appropriate conditions. If a positive signal is observed both of sites of the positive control oligo and the negative control oligo, hybridization was performed under appropriate conditions.

The kit of the present invention contains a substrate on which oligonucleotides are immobilized. In addition, the kit of the present invention may contain primers for preparing a nucleic acid probe or labelled nucleic acid probe, a buffer solution, a reagent for hybridization such as an enzyme conjugate recognizing a hapten and so forth.

### EXAMPLES

Hereafter, the present invention will be explained more specifically with reference to the following examples.

### Example 1: Synthesis of oligonucleotide

An oligonucleotide having an amino group or a hydroxyl group at 5' end was synthesized by using an oligonucleotide synthesizer (Perkin-Elmer Applied Biosystems) in a conventional manner. The synthesized oligonucleotide was deprotected and then dried. The dried oligonucleotide was dissolved in 10 mM Tris-HCl (pH 7.5) and 1 mM EDTA buffer to prepare an oligonucleotide solution of 100 pmol/µl. This synthesis method can be similarly employed in any oligonucleotide which is used as the capture oligo. The nucleotide sequence of the synthetic oligonucleotides are shown as SEQ ID NOS: 1-145 and 160-203 in SEQUENCE LISTING.

### Example 2: Spotting of oligonucleotide to substrate (in case in which oligonucleotide having amino group at 5' end is used)

10 µl of a microspotting solution (TeleChem International Inc.) was mixed with 10 µl of a solution of an oligonucleotide having an amino group at 5' end. The mixture was put on multiple sites on a microtiter plate (Greiner Labotechnik Ltd). A silanized slide glass (Matsunami Glass Inc., Ltd.) was placed at a predetermined position of the spotting machine and the spotting machine was operated. After the spotting was completed, the slide glass was exposed to vapor from hot water for a few seconds and then irradiated with UV light at 30 mJ. The slide glass was again exposed to water vapor for a few seconds and then placed on a hot plate to remove the moisture. The slide glass was rinsed with 0.1% sodium dodecyl sulfate solution and further with distilled water. The slide glass was immersed in 100 mM Tris-HCl, 100 mM NaCl, 0.1% Triton X-100 (pH 7.5) containing 3% BSA (bovine serum albumin) for 30 minutes at room temperature for blocking. Then, the slide glass was dried at room temperature and rinsed with 10 mM Tris-HCl (pH7.5), 1 mM EDTA buffer. The slide glass was again dried at room temperature, and stored in the dried state in a cool and dark place until it was used.

### Example 3: Spotting of oligonucleotide to substrate (in case in which oligonucleotide having hydroxyl group at 5' end is used)

10 µl of a spotting solution (2 M sodium chloride solution) was mixed with 10 µl of a solution of an oligonucleotide having a hydroxyl group at 5' end, and placed on multiple sites on a microtiter plate (Greiner Labotechnik Ltd). A slide glass having carbodiimide groups was placed at a predetermined position of the spotting machine and the spotting machine was operated. After the spotting was completed, the slide glass was put in a dryer set at 37 °C for 30 minutes. The slide glass was immersed in 100 mM Tris-HCl (pH 7.5), 100 mM NaCl, 0.1% Triton X-100 containing 3% BSA at room temperature for 30 minutes for blocking. Then, the slide glass was dried at room temperature and rinsed with 10 mM Tris-HCl (pH7.5), 1 mM EDTA buffer. The slide glass was again dried at room temperature, and stored in the dried state in a cool and dark place until it was used.

### Example 4: Preparation of nucleic acid probe

Bacterial cells were isolated by alkali treatment from specimen according to the standard method presented by U.S. Department of Health and Human Services. Then, genome of the bacterial cells was disrupted by using the method of Jacobs et al., and DNA was extracted (24, 25).

In the first-stage PCR amplification, 1 unit of z-Taq polymerase (TAKARA SHUZO CO., LTD.), 1 µl of 100 pmol/µl 5' end primary primer, 1 µl of 100 pmol/µl 3' end primary primer, 2 pl of a buffer solution for × 10 reaction, 2 µl of 2.5 mM dNTP(Gibco BRL), and 1 µl of 10 ng/µl genome DNA were placed in a 0.2-ml tube and added with sterilized distilled water to a total volume of 20 µl. The tube was set in a thermal cycler (PTC-200, MJ Research Inc.) and a program was operated such that a heating cycle comprising steps of 1) 98°C for 2 minutes; 2) 98°C for 5 seconds; 3) 55°C for 5 seconds; 4) 72°C for 10 seconds; and 5) 72°C for 2 minutes should be performed, where the steps 2) to 4) were repeated 50 times.

In the second-stage PCR amplification, 1 unit of z-Taq polymerase (TAKARA SHUZO CO., LTD.), 1 µl of 100 pmol/pl 5' end secondary primer, 1 µl of 3' end secondary primer of which 5' end was added with biotin, 2 µl of a buffer for ×10 reaction, 2 µl of 2.5 mM dNTP (Gibco BRL), 1 µl of the first PCR solution were placed into a 0.2-ml tube and added with sterilized distilled water to a total volume of 20 µl. The tube was placed in a thermal cycler (PTC-200, MJ Research Inc.) and a program was operated such that a heating cycle comprising steps of 1) 98°C for 2 minutes; 2) 98°C for 5 seconds; 3) 55°C for 5 seconds; 4) 72°C for 10 seconds; and 5) 72°C for 2 minutes should be performed, wherein the steps 2) to 4) were repeated 50 times.

The combinations of the primary primer and the secondary primer employed in the above are shown in Table 3.

In this example, agarose gel electrophoresis described below was carried out as a confirmation test. However, such an agarose gel electrophoresis is not required in differentiation on actual clinical sites.

After the probe synthesis reaction was completed, 1 µl of the reaction mixture was taken and mixed with 1 µl of 6x electrophoresis dye (30% glycerol, 0.25% bromophenol blue, 0.25% xylenecyanol) and 4 µl of 10 mM Tris-HCl (pH 7.5), 1 mM EDTA buffer. After the mixing, the mixture was subjected to electrophoresis on 2% agarose gel at 100 V for 25 minutes. After the electrophoresis, the gel was immersed in distillated water containing ethidium bromide (0.5 µg/ml) for 15 minutes and photographed by illuminating UV light.

### Example 5: Hybridization

5 µl of each nucleic acid probe prepared in Example 3 was taken and placed in a 1.5-ml volume tube, added with 20 µl of UniHyb Hybridization Solution (TeleChem International Inc.) and mixed to prepare a hybridization solution. This solution was subjected to a heat treatment at 100°C for 10 minutes and then immersed in an ice bath for 5 minutes. Then, 10 µl of this nucleic acid probe solution was placed on a substrate on which each oligonucleotide prepared in Example 2 and Example 3 was spotted and covered with a cover glass. The substrate was placed in a hybricasette device (TeleChem international Inc.), immersed in a bath at 42 °C and left for 1 hour with light shielding. The substrate was then taken out from the hybricasette device, and immersed in 5×SSC (0.75 M NaCl, 0.075 M sodium citrate) at 4 °C to remove the cover glass. The substrate was immersed in 5×SSC at 4°C for 30 minutes, and this process was repeated twice. The substrate was then rinsed twice with 3 M tetramethylammonium chloride solution at room temperature, and further rinsed twice with 3 M tetramethylammonium chloride solution at 45°C. Finally, the substrate was transferred to a container containing 2×SSC.

### Example 6: Detection of chemical color development

The substrate was taken out from the container and moisture on the portions on which oligonucleotide had not been immobilized was wiped off with a paper towel. 200 µl of "Block Ace" solution (Dainippon Pharmaceutical Co., Ltd.) was put on the portions at which oligonucleotide had been immobilized. The substrate was left for 30 minutes at room temperature. Thereafter, the solution was sucked up by a dispenser. 200 µl of a solution obtained by mixing 5 ml of TBST solution (50 mM Tris-HCl (pH7.6), 0.15M NaCl, 0.05% Tween 20), a drop of avidin DH and a drop of biotinylated peroxidase (Vector Laboratories, Inc.) was placed on the substrate at the sites where nucleotide had been immobilized. The substrate was left for 30 minutes at room temperature. The solution was sucked up by a dispenser and the substrate was transferred into a container containing a TBST solution. The substrate was washed with the solution for 5 minutes at room temperature while allowing penetration of the solution. The solution in the container was discarded and fresh TBST solution was added to wash the substrate with the solution for 5 minutes at room temperature while allowing penetration of the solution. The substrate was taken out from the container and moisture on the sites on which oligonucleotide had not been immobilized was wiped off with a paper towel. 200 µl of TMB solution (Vector Laboratories, Inc.) was placed on the sited at which oligonucleotide had been immobilized. The substrate was left for 20 minutes at room temperature to allow the reaction. Then, the substrate was washed with deionized water to stop the enzyme reaction.

### Example 7: Determination of drug resistance

After the detection of chemical color development, images were stored as TIFF images in a computer by using a scanner for OA (NEC Multireader 600SR) and Photoshop Ver. 5.0 (Adobe Systems Co., Ltd.). Pigmentation observed in these images, which was formed by the chemical color development, indicates that the oligonucleotide immobilized on the substrate (capture oligo) and the nucleic probe in the test sample are perfectly complementary to each other, forming a duplex.

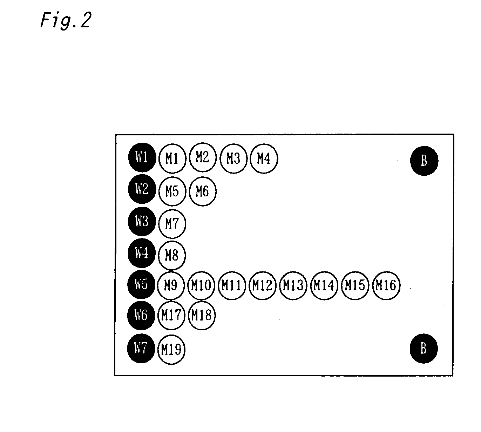

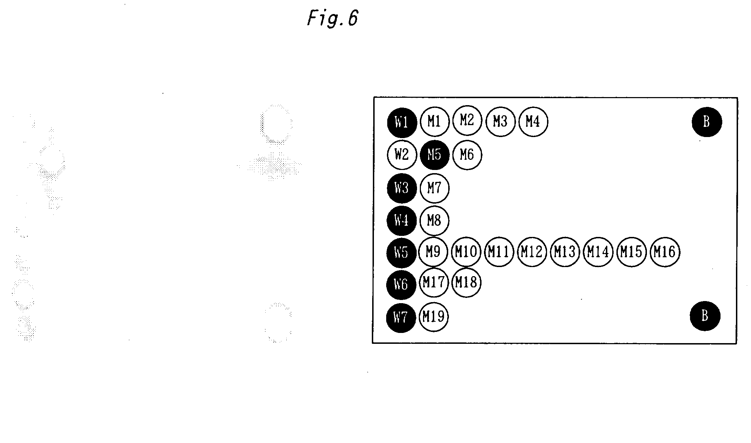

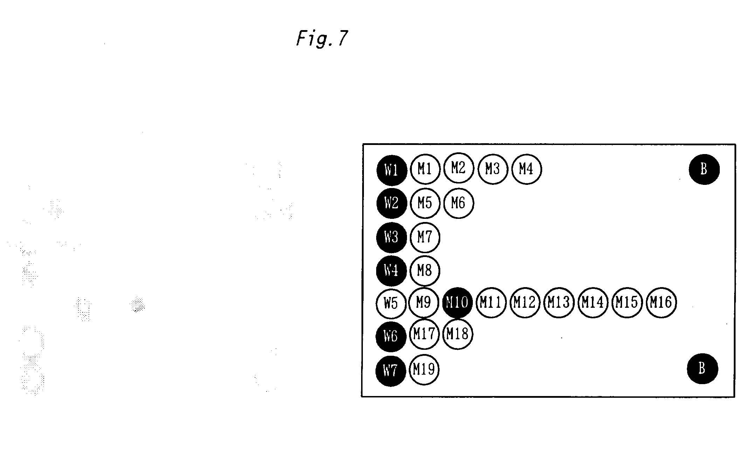

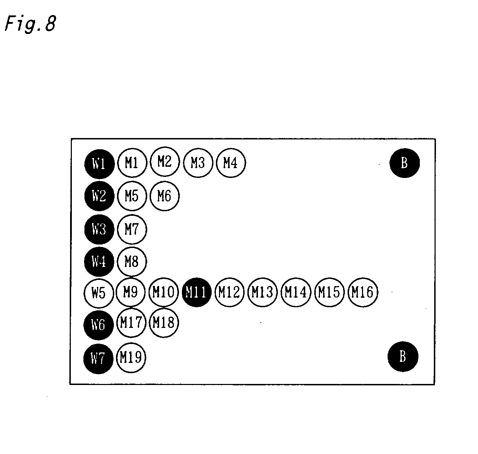

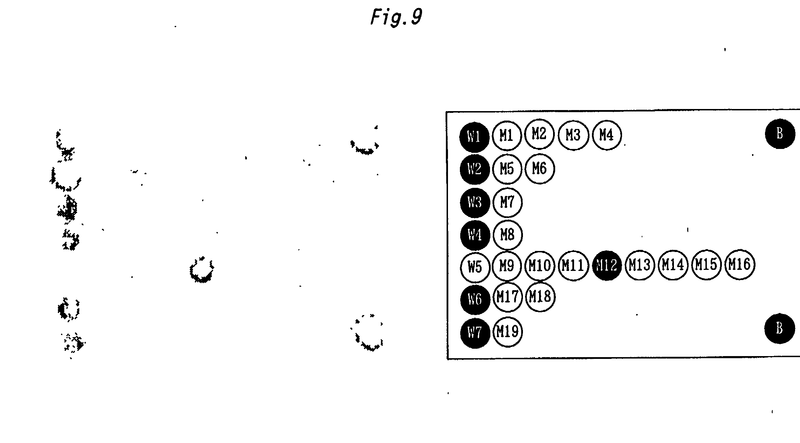

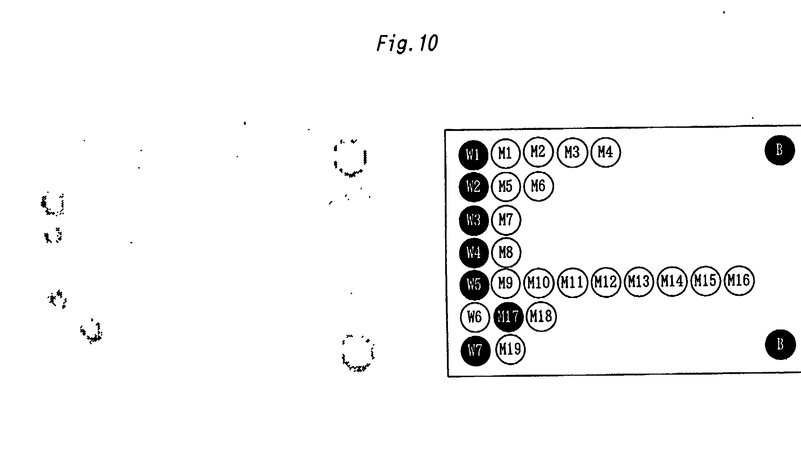

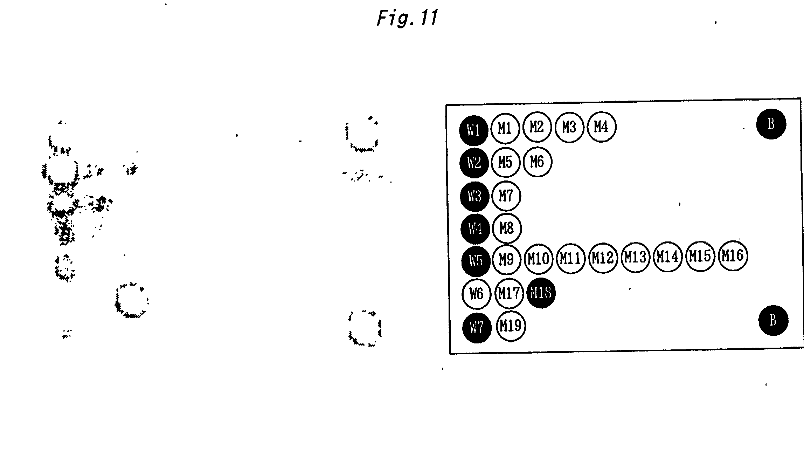

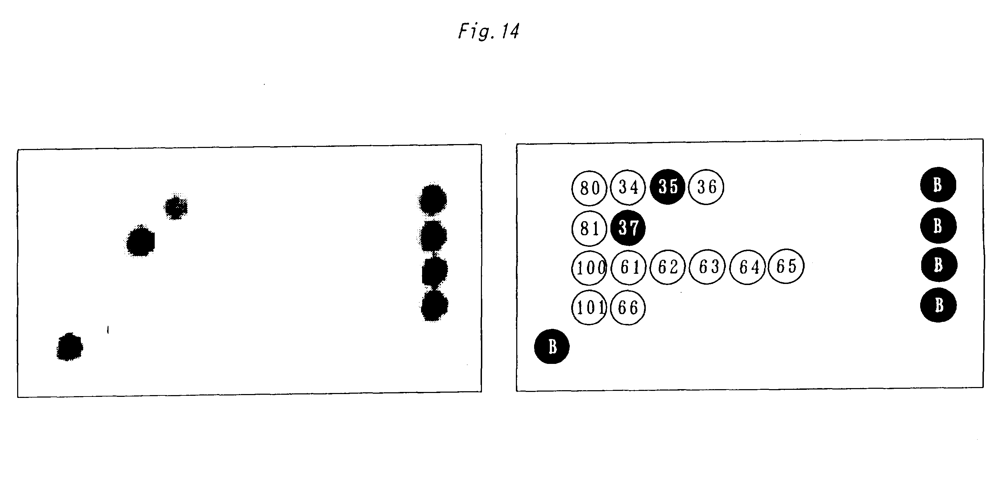

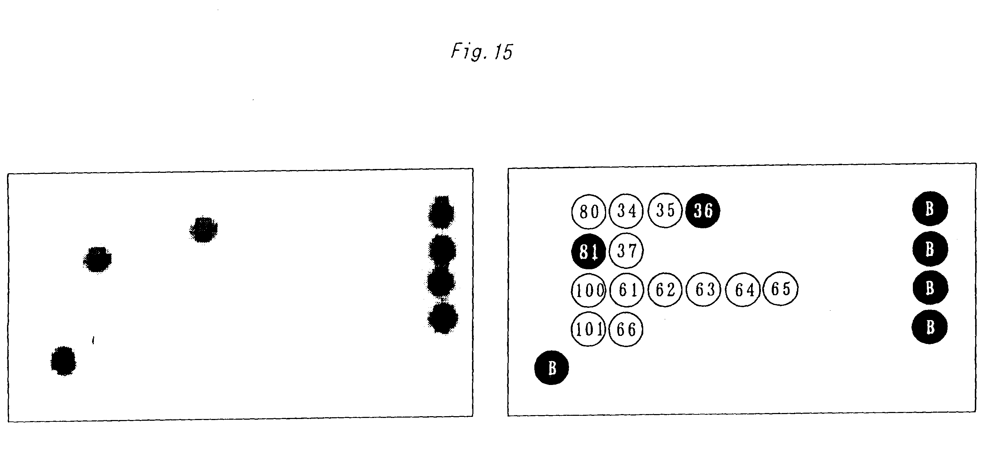

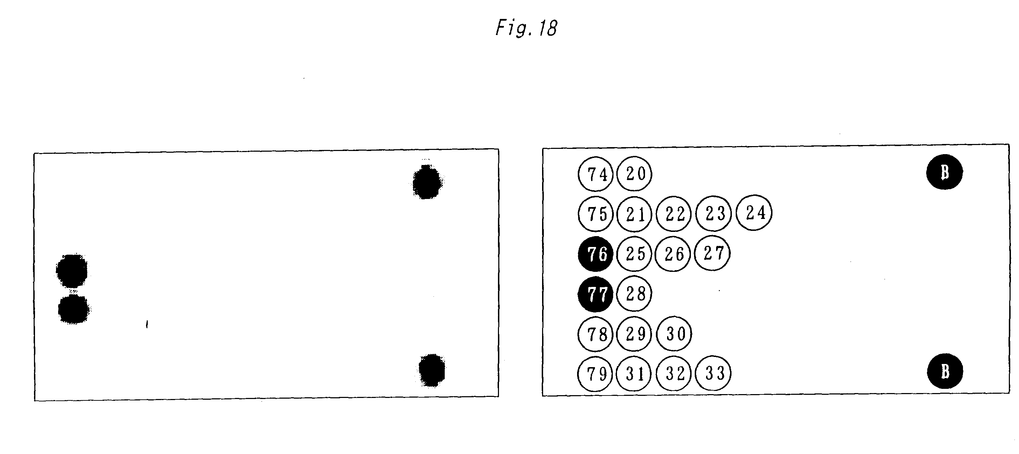

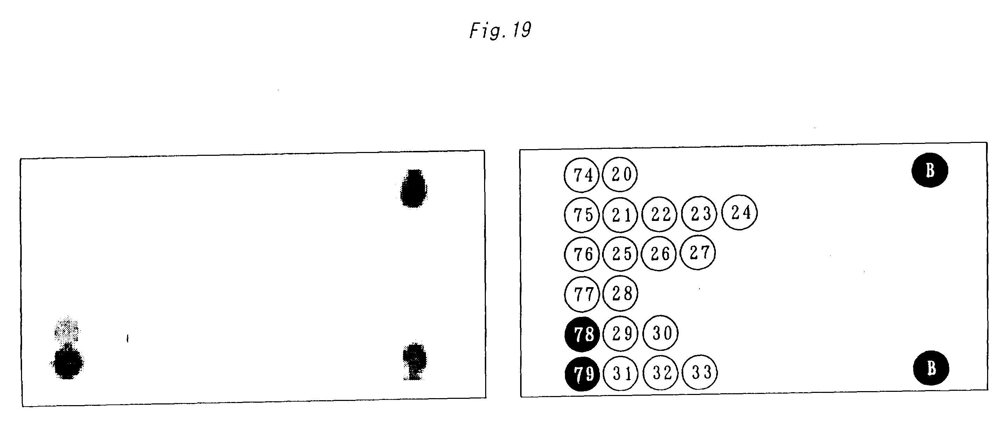

With respect to the arrangement of each drug (5 drugs in total) shown in Fig. 1, the test results of differentiation of each drug resistance determined are shown in Fig. 2 to Fig. 21. Determination using capture oligos for other resistance can also be performed in a similar manner.

The numbers in the photographs each correspond to the sequence numbers. When pigmentation is observed in for all of the capture oligos 67-73 but not observed for any of the capture oligos 1-19, it is diagnosed that the nucleic acid probe derived from that particular reagent DNA is susceptible to rifampicin. On the other hand, when pigmentation is not observed for any of the capture oligos 67-73 derived from the wild-type tubercle bacilli, but pigmentation is observed for at least one of the capture oligos 1-19 which contain a mutation sequence, it is diagnosed that the nucleic acid probe derived from it is resistant to rifampicin. In a case of kanamycin, 80 and 81 correspond to wild-type sequences and 34-37 correspond to mutant type sequences. In a case of streptomycin, 74-79 correspond to wild-type sequences and 20-33 correspond to mutant type sequences. In a case of ethambutol, 100 and 101 correspond to wild-type sequences and 61-66 correspond to mutant type sequences. In a case of isoniazid, 82-99 correspond to wild-type sequences and 38-60 correspond to mutant type sequences.

Figs. 2-21 show experimental results obtained from 21 clinically isolated samples.

In the case shown in Fig. 2, pigmentation was observed in all of 67-73 but not observed in any of 1-19, indicating that the samples were susceptible to rifampicin.

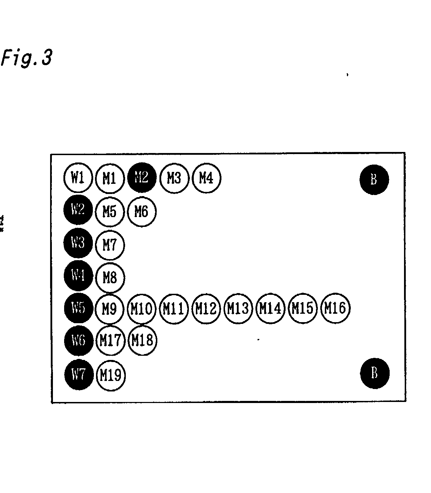

In the case shown in Fig. 3, pigmentation was observed in all of 67-73 expect for W1 and observed in only 1 among 1-19, indicating that the samples were susceptible to rifampicin.

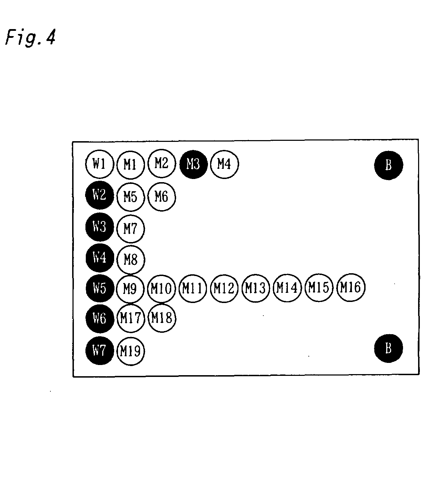

In the case shown in Fig. 4, pigmentation was observed in all of 67-73 expect for 67 and observed in only 3 among 1-19, indicating that the samples were susceptible to rifampicin.

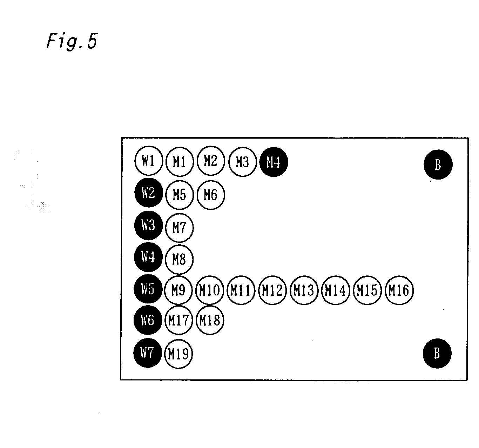

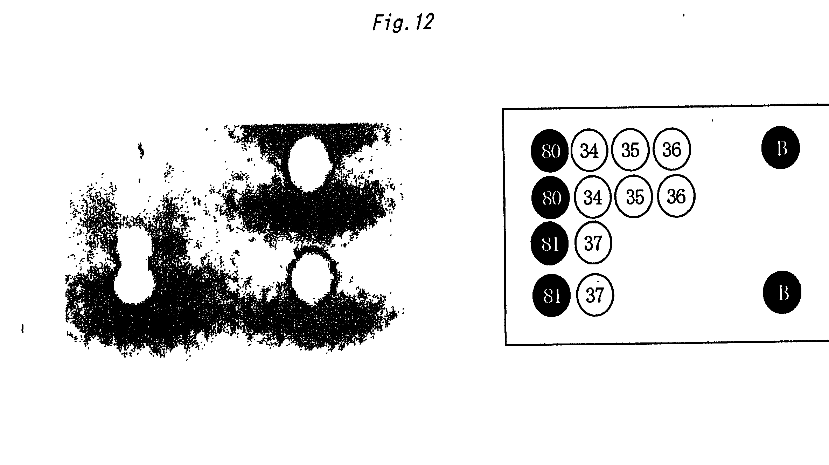

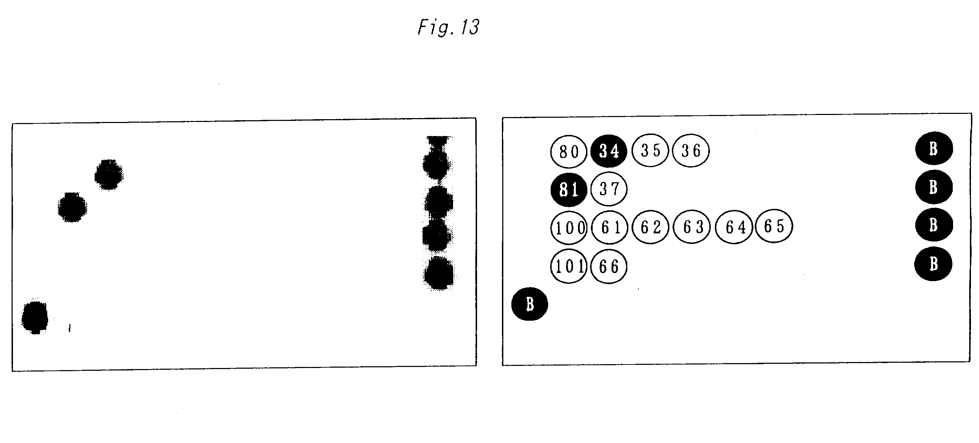

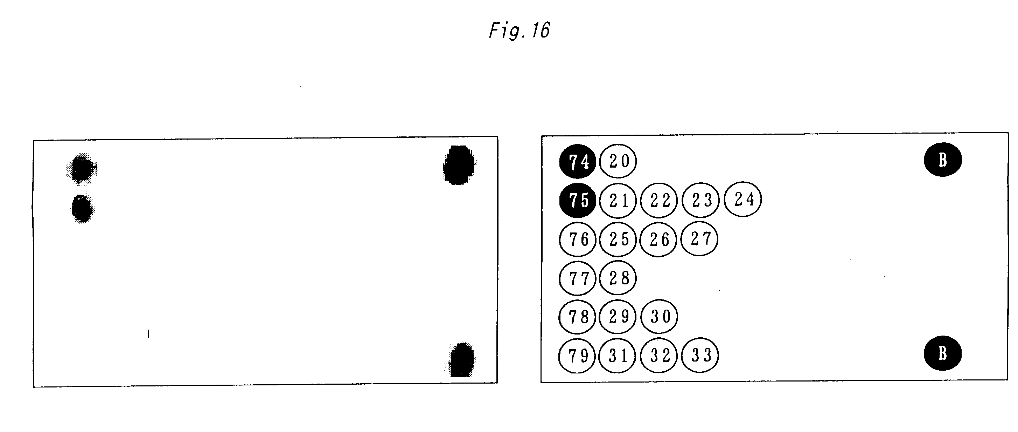

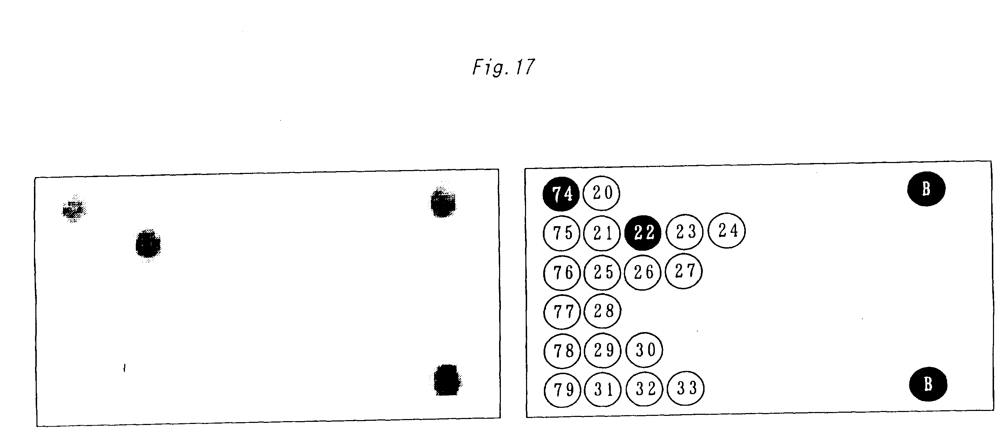

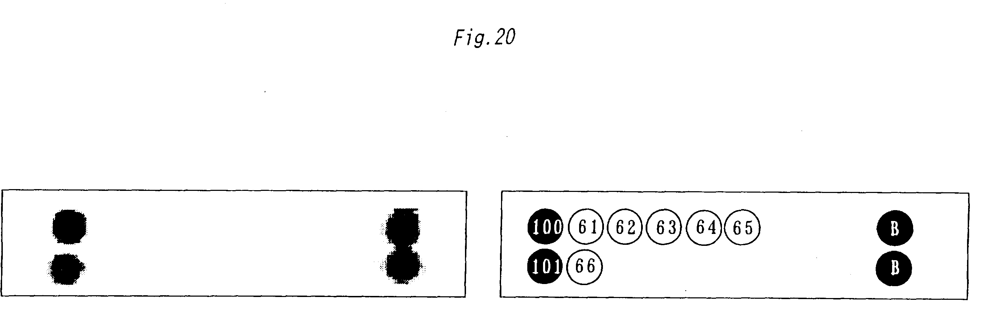

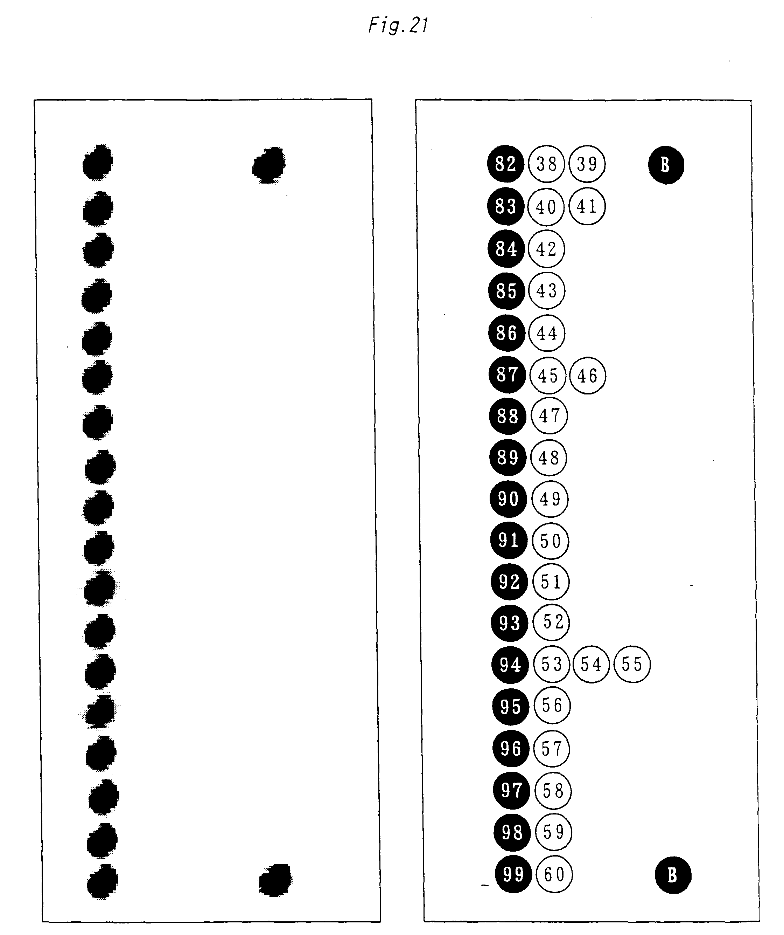

In Figs. 5-11, as pigmentation was observed only in 4, 5, 10, 11, 12, 17 and 18, respectively, and not or hardly observed in the corresponding wild-type sequences (68, 71 and 72), it can be determined that the samples were resistant to rifampicin. In Fig. 12, pigmentation was observed only in 80 and 81 as the wild-type sequences and not observed in the corresponding mutant type sequences. Therefore, it can be determined that the samples were susceptible to kanamycin. In Figs. 13-15, pigmentation was observed only in 34, 35, 37 and 36, and not observed in the corresponding wild-type sequences (80 and 81), indicating that the sample was resistant to kanamycin. In Figs. 16-19, determination was carried out by using sequences derived from sample DNA amplified by the primers of SEQ ID NOS: 108 and 109, and 110, 111, 112 and 113 as probes. In the cases of Figs. 16, 18 and 19, it can be determined that the samples were susceptible to streptomycin, because pigmentation was observed only in 74-79. On the other hand, in the cases of Figs. 17, 18 and 19, it can be determined that the samples were resistant to streptomycin, because pigmentation was observed in 22 but not observed in the wild-type sequence (75). In the aforementioned figures, determination is carried out separately in each case. However, determination may be carried out simultaneously by mixing in advance the sequences amplified by the aforementioned primers 108-113. In Fig. 20, it is determined that the sample is susceptible to ethambutol because pigmentation was observed only in 100 and 101 as the wild-type sequences and was not observed in the corresponding mutant type sequences. In the case of Fig. 21, it is determined that the sample is susceptible to isoniazid because pigmentation was observed in 82-99 but not observed in the corresponding mutation sequences (38-60). These image data was obtained by storing the images of the substrate within the area of 0.24 cm² (0.4 × 0.6 cm) in a computer by a scanner and enlarging the images.

In addition, it is also possible to effect the determination by opening a TIFF image with SCNImage (Scion Corporation) and measuring strength of each spot. The results obtained by carrying out the measurement in such a manner for the cases of Figs. 3-11 are summarized in Table 4. In Table 4, it can be determined that the sample was resistant to rifampicin because the strength values of the mutant type sequences are larger than those of the corresponding wild-type sequences.

**Table 4:**

| The measurement results in Figs. 3-11 | | | | |
|---|---|---|---|---|
| Figure | Wild-type | Measured value | Mutant Type | Measured value |
| 3 | W1 | 0.00 | m2 | 0.24 |
| 4 | W1 | 0.01 | m3 | 0.11 |
| 5 | W1 | 0.01 | m4 | 0.22 |
| 6 | W2 | 0.02 | m5 | 0.32 |
| 7 | W5 | 0.07 | M10 | 0.29 |
| 8 | W5 | 0.04 | M11 | 0.20 |
| 9 | W5 | 0.00 | M12 | 0.34 |
| 10 | W6 | 0.01 | M17 | 0.37 |
| 11 | W6 | 0.08 | M18 | 0.58 |

### Example 8: Determination of drug resistance

In order to demonstrate that the concept of the present invention can be realized with sequences shown as SEQ ID NOS: 1-66 of which 5' end and/or 3' end are shortened or extended, the oligomers shown in the correlation table (Table 5) was synthesized according to the method described in Example 1.

**Table 5:**

| Capture correlation table | | | |
|---|---|---|---|
| Sequence No. | Corresponding sequence (No.) | Sequence No. | Corresponding sequence (No.) |
| 166 | 67 | 185 | 17 |
| 167 | 1 | 186 | 18 |
| 168 | 3 | 187 | 73 |
| 169 | 4 | 188 | 74 |
| 170 | 5 | 189 | 22 |
| 171 | 6 | 190 | 25 |
| 172 | 69 | 191 | 26 |
| 173 | 70 | 192 | 27 |
| 174 | 8 | 193 | 77 |
| 175 | 71 | 194 | 78 |
| 176 | 9 | 195 | 31 |
| 177 | 10 | 196 | 32 |
| 178 | 11 | 197 | 33 |
| 179 | 12 | 198 | 80 |
| 180 | 13 | 199 | 34 |
| 181 | 14 | 200 | 35 |
| 182 | 15 | 201 | 36 |
| 183 | 16 | 202 | 81 |
| 184 | 72 | 203 | 37 |

The SEQ ID NOS: 166-203 were spotted on the substrate in a manner similar to Example 2 and Example 3. In addition, the nucleic probes were prepared in a manner similar to Example 4. Further, hybridization was performed in a manner similar to that of Example 5 and detection of chemical color development was carried out in a manner similar to that of Example 6. Determination of drug resistance was carried out in a manner described in Example 7.

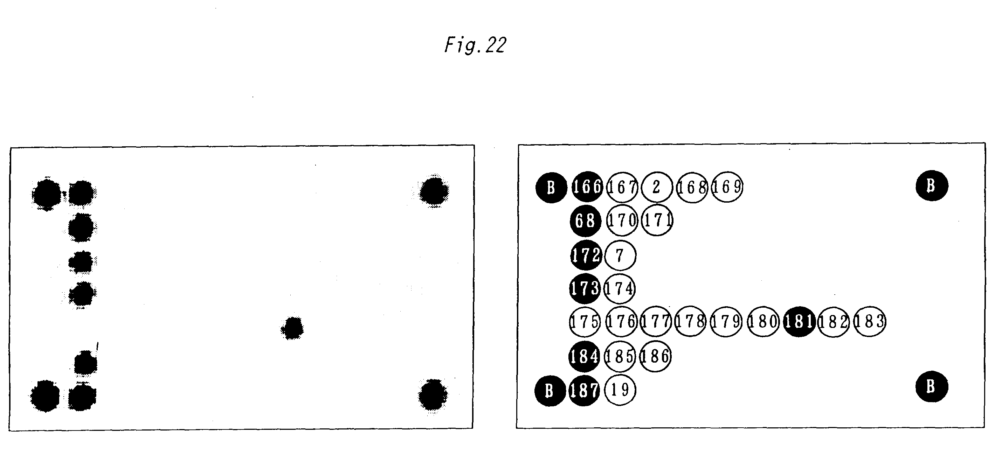

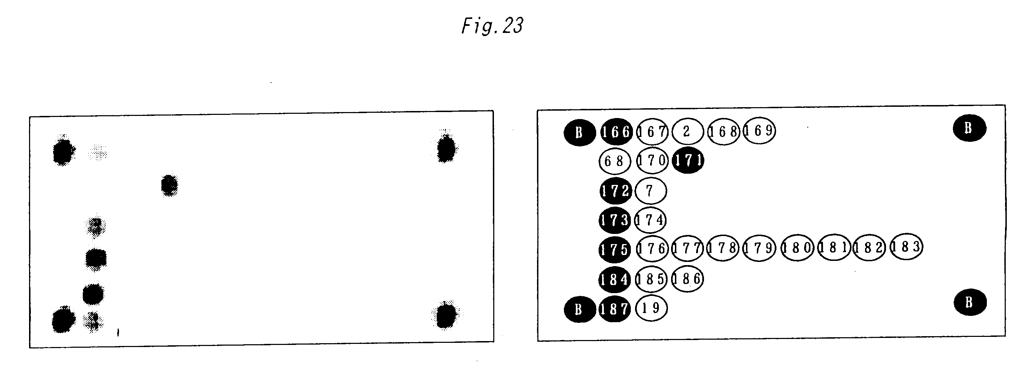

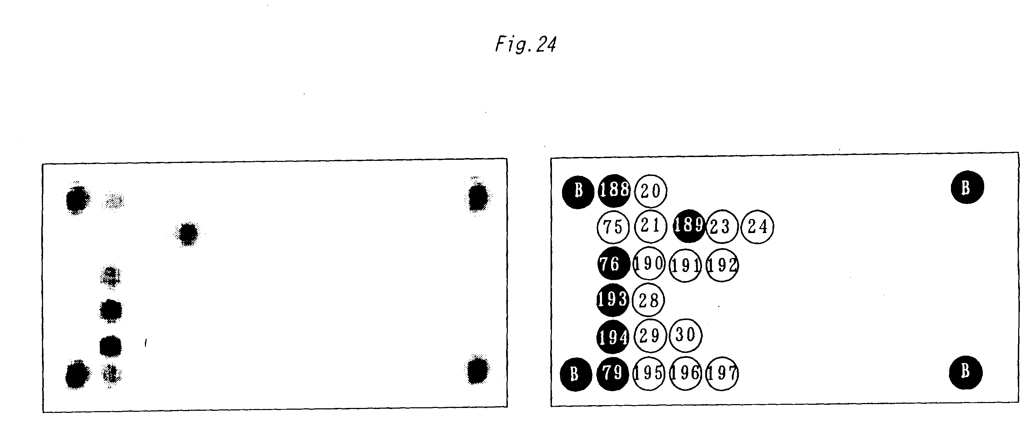

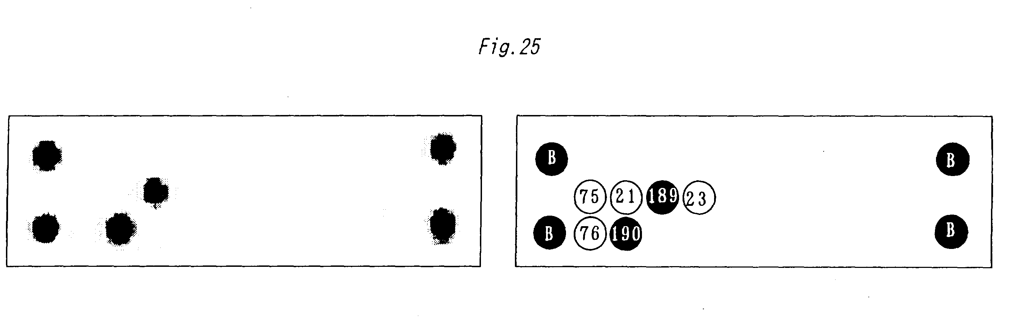

Figs. 22 and 23 show the determination results with respect to resistance to rifampicin. Fig. 24 shows the determination results with respect to resistance to streptomycin. Fig. 25 shows the determination results with respect to resistance to kanamycin. From the determination results shown in Figs. 22-25, it can be seen that the present invention can be practiced with sequences of SEQ ID NOS: 1-66 of which 5' end and/or 3' end are shortened or extended.

### REFERENCES

1. Suzuki, S. et al, "Drug resistance test of tubercle bacillus: Genes responsible for drug resistance and possibility of application to quick differentiation", Rinsho to Biseibutsu (Clinic and Microorganism), 125:795-801, 1998
2. Hirano, K. et al., "Resistance to antituberculosis drugs in Japan" Tuber. Lung. Dis., 77:130-135, 1996
3. Mori, T., "Current status and remedy of tuberculosis in Japan", Igaku no Ayumi (Proceedings of Medicine), 189:869-872, 1999
4. Tsuyuguchi, I., "Tuberculosis - From infection to onset", Igaku no Ayumi (Proceedings of Medicine), 189:873-876, 1999
5. Abe, C., "Progress of detection methods of tubercle bacillus", Igaku no Ayumi (Proceedings of Medicine), 189:877-880, 1999
6. Abe, C. et al., "Detection of micobacterium tuberculosis in clinical specimens by polymerase chain reaction and Gen-Probe Amplified Mycobacterium Tuberculosis Direct Test", J. Clin. Microbial., 31:3270-3274, 1993
7. Abe, C. et al., "Cooperative research on MTD evaluation for quick detection of tubercle bacillus", Kekkaku (Tuberculosis), 70:467-472, 1993
8. Telenti, A. et al., "Detection of rifampicin-resistance mutations in Mycobacterium tuberculosis", Lancet, 341:647-650, 1993
9. Suzuki, Y. et al., "Mutations in rpoB gene of rifampicin resistant clinical isolates of Mycobacterium tuberculosis in Japan", Journal of Infection Diseases, 69:413, 1995
10. Finken, M. et al., "Molecular basis of streptomycin resistance in Mycobacterium tuberculosis: alternation of the ribosomal protein S12 gene and point mutations within a function 16S ribosomal RNA psudoknot", Mol. Microbiol., 9:1293, 1993
11. Katsukawa. C. et al., "Characterization of the rpsL and rrs genes of streptomycin-resistant clinical isolates of Mycrobacterium tuberculosis in Japan" J. Appl. Microbiol., 9:1239, 1993
12. Sreevastan, S. et al., "Ethambutol registance in Mycrobacterium tuberculosis: Clinical role of embB mutations", Antimicrob. Chemothera. 41:1677, 1997
13. Banerjee, A. et al., "ihnA, a gene encoding a target for isoniazid and ethinamide in Mycobacterium tuberculosis", Science 263:227, 1994
14. Hirano, K. et al., "Mutation in pncA is a major mechanism of pyrazinamide resistance in Mycobactrium tuberculosis", Tuber. Lung Dis. 78:117, 1997
15. Ono, H. et al., "2. Mechanism for emergence of resistant bacteria", Kekkaku, 73:657-663, 1998
16. Suzuki. Y. et al., "Detection of Kanamycin-resistant Mycobacterium tuberculosis by identifying mutations in the 16S rRNA gene", J. Clin. Microbiol., 36:1220-1225
17. Telenti, A. et al., Antimicrob. Chemother., 37:2054-2058, 1993
18. Nair, J. et al., Mol. Microbiol., 10:521-527, 1993
19. Piatek, A. S. et al., "Molecular beacon sequence analysis for detecting drug-resistance in Mycrobacterial tuberculosis", Nat. Biotecnol., 16:359, 1998
20. Gingeras, T. A. et al.,"CHIP-BASED SPECIATION AND PHENOTYPIC CHARCTERIZATION OF MICROORGANISMS", WO97/29212
21. Zang, Y. et al., "Molecular basis for the exquisite sensitivity of Mycrobacterium tuberculosis to isoniazide", Proc. Natl. Sci. USA, 93:13212, 1996
22. Taniguchi, H. et al., "Molecular analysis of kanamycin and viomycin resistance in Mycrobacterium tuberculosis by use of the conjugation system", J. Bacteteriol., 179:4795, 1997
23. Telenti. A. et al., "The emb operon, a gene cluster of Mycrobacterium tuberculosis involved in resistance to ethambutol", Nat. Med., 3:567, 1997
24. Jacobs, W. R. Jr. et al., "Genetic system for Mycobacteria", Methods in Enzymology, 204: 537, 1991
25. Kent, P.T. et al., Mycobacteriology. A guide for the Level III laboratory. pp 31, U.S. Department of Health and Human Services. Public Health Service, Centers for Disease Control, 1985
26. Davies, P. D. O., Multi-drug resistant tuberculosis., Priory Lodge Education Ltd, 1999
27. CDEE,MARK et al. "ARRAYS OF NUVLEIC ACID PROBES ON BIOLOGOCAL CHIPS" W095/11995
28. CHEE, MARK, et al "ARRAYS OF NUCLEIC ACID PROBES ON BIOLOGICAL CHIPS" , USP 5,837,832

## Claims

1. A kit for diagnosis of tubercle bacilli containing a substrate, on which immobilized through a covalent bond is an oligonucleotide which is derived from a gene on tubercle bacilli genome that is responsible for drug resistance for one or more drugs employed in tuberculosis treatment and synthesized based on nucleotide sequences obtained from a wild-type tubercle bacilli susceptible to the drug or drugs and a mutant type tubercle bacilli resistant to at least one of the drugs, wherein differentiation of drug resistance of tubercle bacilli or determination of infection with tubercle bacilli is attained by hybridization of the oligonucleotide with a nucleic acid derived from a microorganism to be tested.

2. The kit according to claim 1, wherein a compound having a carbodiimide group or an isocyanate group is coated on a surface of the substrate, and the covalent bond is formed as a result of a reaction between the carbodiimide group or the isocyanate group and the oligonucleotide or an amino group of a linker added to an end of the oligonucleotide.

3. The kit according to claim 1, wherein the oligonucleotide is immobilized on an area of the substrate surface having a diameter of 10-1000 µm.

4. The kit according to claim 1, wherein the gene that is responsible for the drug resistance consists of one or more of genes selected from the genes that are responsible for resistance to rifampicin, streptomycin, kanamycin, isoniazid or ethambutol.

5. The kit according to claim 4, wherein the gene that is responsible for the drug resistance is the gene that are responsible for resistance to rifampicin and isoniazid.

6. The kit according to claim 4, wherein the gene that is responsible for the drug resistance consists of all of the genes that are responsible for resistance to rifampicin, streptomycin, kanamycin, isoniazid and ethambutol, respectively.

7. An oligonucleotide which is derived from a mutant type gene having a point mutation responsible for the drug resistance contained in a mutant type tubercle bacilli which is resistant to any one of drugs selected from rifampicin, streptomycin, kanamycin, isoniazid and ethambutol, and designed and synthesized so as to have a length of 12-24 nucleotides containing the mutation.

8. The oligonucleotide according to claim 7, which is derived from a sequence of a rpoB gene of a mutant type tubercle bacilli resistant to rifampicin which contains the mutation and contains any one of the nucleotide sequences shown as SEQ ID NOS: 1-19.

9. The oligonucleotide according to claim 7, wherein the oligonucleotide is a nucleotide sequence obtained by extending or shortening any one of the nucleotide sequences shown as SEQ ID NOS: 1-19 at the 5' end and/or the 3' end with or for a gene sequence of a wild-type tubercle bacilli of which position on the tubercle bacilli genome corresponds to each of the nucleotide sequences, and the mutation contained in each of the original nucleotide sequences is not substituted.

10. The oligonucleotide according to claim 7, wherein the oligonucleotide contains one of the nucleotide sequences shown as SEQ ID NOS: 20-28 derived from a sequence containing the mutation of an rrs gene of a mutant type tubercle bacilli resistant to streptomycin or one of nucleotide sequences shown as SEQ ID NOS: 29-33 derived from a sequence containing the mutation of an rpsL gene of a mutant type tubercle bacilli resistant to streptomycin.

11. The oligonucleotide according to claim 7, wherein the oligonucleotide is a nucleotide sequence obtained by extending or shortening any one of the nucleotide sequences shown as SEQ ID NOS: 20-33 as a base sequence at the 5' end and/or the 3' end with or for a gene sequence of a wild-type tubercle bacilli of which position on the tubercle bacilli genome corresponds to each of the nucleotide sequences, and the mutation contained in each of the original nucleotide sequences is not substituted.

12. The oligonucleotide according to claim 7, which is derived from a sequence which contains the mutation of an rrs gene of a mutant type tubercle bacilli resistant to kanamycin and contains one of the nucleotide sequences shown as SEQ ID NOS: 34-37.

13. The oligonucleotide according to claim 7, wherein the oligonucleotide is a nucleotide sequence obtained by extending or shortening any one of the nucleotide sequences shown as SEQ ID NOS: 34-37 as a base sequence at the 5' end and/or the 3' end with or for a gene sequence of a wild-type tubercle bacilli of which position on the tubercle bacilli genome corresponds to each of the nucleotide sequences, and the mutation contained in each of the original nucleotide sequences is not substituted.

14. The oligonucleotide according to claim 7, wherein the oligonucleotide contains one of the nucleotide sequences shown as SEQ ID NOS: 38-41 derived from a sequence containing the mutation of an inhA gene of a mutant type tubercle bacilli resistant to isoniazid or one of the nucleotide sequences shown as SEQ ID NOS: 42-60 derived from a sequence containing the mutation of a katG gene of a mutant type tubercle bacilli resistant to isoniazid.

15. The oligonucleotide according to claim 7, wherein the oligonucleotide is a nucleotide sequence obtained by extending or shortening any one of the nucleotide sequences shown as SEQ ID NOS: 38-60 as a base sequence at the 5' end and/or the 3' end with or for a gene sequence of a wild-type tubercle bacilli of which position on the tubercle bacilli genome corresponds to each of the nucleotide sequences, and the mutation contained in each of the original nucleotide sequences is not substituted.

16. The oligonucleotide according to claim 7, which is derived from a sequence containing the mutation of an embB gene of a mutant type tubercle bacilli resistant to ethambutol and contains one of the nucleotide sequences shown as SEQ ID NOS: 61-66.

17. The oligonucleotide according to claim 7, wherein the oligonucleotide is a nucleotide sequence obtained by extending or shortening any one of the nucleotide sequences shown as SEQ ID NOS: 61-66 as a base sequence at the 5' end and/or the 3' end with or for a gene sequence of a wild-type tubercle bacilli of which position on the tubercle bacilli genome corresponds to each of the nucleotide sequences, and the mutation contained in each of the original nucleotide sequences is not substituted.

18. An oligonucleotide which is derived from a wild-type gene contained in a wild-type tubercle bacilli which is susceptible to rifampicin, streptomycin, kanamycin, isoniazid and ethambutol, obtained from a gene sequence of which position on the tubercle bacilli genome corresponds to that of a nucleotide sequence of an oligonucleotide contained in a mutant type gene responsible for resistance to the drugs, and designed and synthesized so as to have a length of 12-24 nucleotides.

19. The oligonucleotide according to claim 18, which is derived from a rpoB gene which contains no mutation of a wild-type tubercle bacilli, and contains one of the nucleotide sequences shown as SEQ ID NOS: 67-73.

20. The oligonucleotide according to claim 18, which is a nucleotide sequence obtained by extending or shortening any one of the nucleotide sequences shown as SEQ ID NOS: 67-73 as a base sequence at the 5' end and/or the 3' end with or for a gene sequence of a wild-type tubercle bacilli of which position on the tubercle bacilli genome corresponds to each of the nucleotide sequences.

21. The oligonucleotide according to claim 18, which contains one of the nucleotide sequences shown as SEQ ID NOS: 74-77 derived from an rrs gene containing no mutation of a wild-type tubercle bacilli or one of the nucleotide sequences shown as SEQ ID NOS: 78-79 derived from an rpsL gene.

22. The oligonucleotide according to claim 18, which is a nucleotide sequence obtained by extending or shortening any one of the nucleotide sequences shown as SEQ ID NOS: 74-79 as a base sequence at the 5' end and/or the 3' end with or for a gene sequence of a wild-type tubercle bacilli of which position on the tubercle bacilli genome corresponds to each of the nucleotide sequences.

23. The oligonucleotide according to claim 18, which is derived from an rrs gene containing no mutation of a wild-type tubercle bacilli, and contains the nucleotide sequence shown as SEQ ID NO: 80 or 81.

24. The oligonucleotide according to claim 18, which is a nucleotide sequence obtained by extending or shortening the nucleotide sequence shown as SEQ ID NO: 80 or 81 as a base sequence at the 5' end and/or the 3' end with or for a gene sequence of a wild-type tubercle bacilli of which position on the tubercle bacilli genome corresponds to each of the nucleotide sequences.

25. The oligonucleotide according to claim 18, which contains the nucleotide sequence shown as SEQ ID NO: 82 or 83 derived from an inhA gene containing no mutation of a wild-type tubercle bacilli or one of the nucleotide sequences shown as SEQ ID NO: 84-99 derived from a katG gene.

26. The oligonucleotide according to claim 18, which is a nucleotide sequence obtained by extending or shortening any one of the nucleotide sequences shown as SEQ ID NOS: 83-99 as a base sequence at the 5' end and/or the 3' end with or for a gene sequence of a wild-type tubercle bacilli of which position on the tubercle bacilli genome corresponds to each of the nucleotide sequences.

27. The oligonucleotide according to claim 18, which is derived from an embB gene contain no mutation of a wild-type tubercle bacilli, and contains the nucleotide sequence shown as SEQ ID NO: 100 or 101.

28. The oligonucleotide according to claim 18, which is a nucleotide sequence obtained by extending or shortening the nucleotide sequence shown as SEQ ID NO: 100 or 101 as a base sequence at the 5' end and/or the 3' end with or for a gene sequence of a wild-type tubercle bacilli of which position on the tubercle bacilli genome corresponds to each of the nucleotide sequences.

29. The kit according to any one of claims 1 to 6, wherein the synthetic oligonucleotide contains a combination of (a) and (b) mentioned below, and positions on tubercle bacilli genome of a nucleotide sequence of the oligonucleotide of (a) and a nucleotide sequence of the oligonucleotide of (b) are correspond to each other:
(a) an oligonucleotide which is derived from a mutant type gene having a point mutation responsible for the drug resistance contained in a mutant type tubercle bacilli which is resistant to any one of drugs selected from rifampicin, streptomycin, kanamycin, isoniazid and ethambutol, and contains
any one of the nucleotide sequences shown as SEQ ID NOS: 1-66; or
a nucleotide sequence which is a nucleotide sequence obtained by extending or shortening any one of the nucleotide sequences at the 5' end and/or the 3' end with or for a gene sequence of a wild-type tubercle bacilli of which position on the tubercle bacilli genome corresponds to each of the nucleotide sequences, and designed so as not to substitute the mutation contained in each of the original nucleotide sequences and to have a length of 12-24 nucleotides, and
(b) an oligonucleotide which is a wild-type gene of a wild-type tubercle bacilli which is susceptible to rifampicin, streptomycin, kanamycin, isoniazid and ethambutol, and contains
any one of the nucleotide sequences shown as SEQ ID NOS: 67-101; or
a nucleotide sequence which is obtained from a gene sequence of which position on the tubercle bacilli genome corresponds to each of the nucleotide sequences of (a), and designed so as to have a length of 12-24 nucleotides.

30. The kit according to claim 29, which is a kit for differentiating resistance to rifampicin and isoniazid, wherein
the oligonucleotide of (a) contains
an oligonucleotide selected from the group consisting of the nucleotide sequences shown as SEQ ID NOS: 1-19 and the group consisting of the nucleotide sequences shown as SEQ ID NOS: 38-60, or
an oligonucleotide which is a nucleotide sequence obtained by extending or shortening a nucleotide sequence of any one of the aforementioned oligonucleotides at the 5' end and/or the 3' end with or for a gene sequence of a wild-type tubercle bacilli of which position on the tubercle bacilli genome corresponds to each of the nucleotide sequences and selected from those derived from the group consisting of the nucleotide sequences shown as SEQ ID NOS: 1-19 and the group consisting of the nucleotide sequences shown as SEQ ID NOS: 38-60, and designed so as not to substitute the mutation contained in each of the original nucleotide sequences and to have a length of 12-24 nucleotides, and
the oligonucleotide of (b) contains
an oligonucleotide selected from the group consisting of the nucleotide sequences shown as SEQ ID NOS: 61-66 and the group consisting of the nucleotide sequences shown as SEQ ID NOS: 82-89, or
an oligonucleotide which is a nucleotide sequence obtained by extending or shortening a nucleotide sequence of any one of the aforementioned oligonucleotides at the 5' end and/or the 3' end with or for a gene sequence of a wild-type tubercle bacilli of which position on the tubercle bacilli genome corresponds to each of the nucleotide sequences and selected from those derived from the group consisting of the nucleotide sequences shown as SEQ ID NOS: 61-66 and the group consisting of the nucleotide sequences shown as SEQ ID NOS: 82-89, and designed so as not to substitute the mutation contained in each of the original nucleotide sequences and to have a length of 12-24 nucleotides.

31. The kit according to claim 29, which is a kit for differentiating resistance to multiple drugs, wherein
the oligonucleotide of (a) contains an oligonucleotide selected from the group consisting of the nucleotide sequences shown as SEQ ID NOS: 1-19, the group consisting of the nucleotide sequences shown as SEQ ID NOS: 20-28, the group consisting of the nucleotide sequences shown as SEQ ID NOS: 29-33, the group consisting of the nucleotide sequences shown as SEQ ID NOS: 34-37, the group consisting of the nucleotide sequences shown as SEQ ID NOS: 38-41, the group consisting of the nucleotide sequences shown as SEQ ID NOS: 42-60 and the group consisting of the nucleotide sequences shown as SEQ ID NOS: 61-66, or
an oligonucleotide which is a nucleotide sequence obtained by extending or shortening a nucleotide sequence of any one of the aforementioned oligonucleotides at the 5' end and/or the 3' end with or for a gene sequence of a wild-type tubercle bacilli of which position on the tubercle bacilli genome corresponds to each of the nucleotide sequences and selected from the group consisting of the nucleotide sequences shown as SEQ ID NOS: 1-19, the group consisting of the nucleotide sequences shown as SEQ ID NOS: 20-28, the group consisting of the nucleotide sequences shown as SEQ ID NOS: 29-33, the group consisting of the nucleotide sequences shown as SEQ ID NOS: 34-37, the group consisting of the nucleotide sequences shown as SEQ ID NOS: 38-41, the group consisting of the nucleotide sequences shown as SEQ ID NOS: 42-60 and the group consisting of the nucleotide sequences shown as SEQ ID NOS: 61-66 and, and designed so as not to substitute the mutation contained in each of the original nucleotide sequences and to have a length of 12-24 nucleotides, and
the oligonucleotide of (b) contains an oligonucleotide selected from the group consisting of the nucleotide sequences shown as SEQ ID NOS: 67-73, the group consisting of the nucleotide sequences shown as SEQ ID NOS: 74-77 and SEQ ID NOS: 78-79, the group consisting of the nucleotide sequences shown as SEQ ID NOS: 80-81, the group consisting of the nucleotide sequences shown as SEQ ID NOS: 82-83, the group consisting of the nucleotide sequences shown as SEQ ID NOS: 84-99 and the group consisting of the nucleotide sequences shown as SEQ ID NOS: 100-101, or
an oligonucleotide which is a nucleotide sequence obtained by extending or shortening an oligonucleotide of any one of the aforementioned nucleotide sequences at the 5' end and/or the 3' end with or for a gene sequence of a wild-type tubercle bacilli of which position on the tubercle bacilli genome corresponds to each of the nucleotide sequences and selected from those derived from the group consisting of the nucleotide sequences shown as SEQ ID NOS: 1-19, the group consisting of the nucleotide sequences shown as SEQ ID NOS: 20-28, the group consisting of the nucleotide sequences shown as SEQ ID NOS: 29-33, the group consisting of the nucleotide sequences shown as SEQ ID NOS: 34-37, the group consisting of the nucleotide sequences shown as SEQ ID NOS: 38-41, the group consisting of the nucleotide sequences shown as SEQ ID NOS: 42-60 and the group consisting of the nucleotide sequences shown as SEQ ID NOS: 61-66, and designed so as not to substitute the mutation contained in each of the original nucleotide sequences and to have a length of 12-24 nucleotides.

32. The kit according to any one of claims 1-6, wherein, in addition to the synthetic oligonucleotide, an oligonucleotide synthesized from those of (c) and (d) is immobilized on the substrate, the oligonucleotide of (c) serves as a positive control specific to tubercle bacilli DNA and the oligonucleotide of (d) serves as a negative control which does not cause sequence-specific hybridization with tubercle bacilli DNA:
(c) an oligonucleotide obtained from a gene region which is commonly contained in a wild-type tubercle bacilli susceptible to a drug and mutant type tubercle bacilli resistant to a drug but is not genetically conserved among *Mycobacterial* species;
(d) an oligonucleotide obtained by substituting one or more nucleotides of a nucleotide sequence of the oligonucleotide of (c) within a range of from one nucleotide to less than 20% of the total number of nucleotides of the nucleotide sequence.

33. The kit according to claim 32, wherein the oligonucleotide of (c) contains the nucleotide sequence shown as SEQ ID NO: 102 derived from an rrs gene.

34. The kit according to claim 32, wherein the oligonucleotide of (c) contains the nucleotide sequence shown as SEQ ID NO: 102 and the oligonucleotide of (d) contains the nucleotide sequence shown as SEQ ID NO: 103.

35. A method for producing a labeled nucleic acid probe used for differentiation of drug resistance of tubercle bacilli or determination of infection with tubercle bacilli using the kit according to claim 1 and derived from a nucleic acid of a microorganism to be tested, which comprises steps of:
extracting DNA from bacterial cells of a microorganism to be tested obtained from the microorganism to be tested separated from sputum or the microorganism to be tested obtained by culture; and
performing nucleic acid amplification by employing the DNA as a template and using a primer labeled with a fluorescent substance or a hapten at the 5' end or 3' end.

36. A nucleic acid probe obtained by the method according to claim 35, which is labeled with a fluorescent substance selected from fluorescein (FITC), Rodamine, phycoerythrin (PE), Texas Red and cyanine fluorescent dyes.

37. A nucleic acid probe obtained by the method according to claim 35, which is labeled with a hapten selected from biotin, digoxigenin (Dig), dinitrophenyl (DNP) and fluorescein.

38. Primers used for the method according to claim 35, which comprises:
a pair of oligonucleotides having the nucleotide sequences shown as SEQ ID NOS: 106 and 107, which correspond to a part of rpoB gene sequence that is responsible for resistance to rifampicin;
a pair of oligonucleotides having the nucleotide sequences shown as SEQ ID NOS: 108 and 109 or the nucleotide sequences shown as SEQ ID NOS: 110 and 111, which correspond to a part of rrs gene sequence that is responsible for resistance to streptomycin;
a pair of oligonucleotides having the nucleotide sequences shown as SEQ ID NOS: 112 and 113, which correspond to a part of rpsL gene sequence that is responsible for resistance to streptomycin;
a pair of oligonucleotides having the nucleotide sequences shown as SEQ ID NOS: 114 and 115, which correspond to a part of rrs gene sequence that is responsible for resistance to kanamycin;
a pair of oligonucleotides having the nucleotide sequences shown as SEQ ID NOS: 116 and 117, which correspond to a part of inhA gene sequence that is responsible for resistance to isoniazid;
a pair of oligonucleotidesr having the nucleotide sequences shown as SEQ ID NOS: 118 and 119, 120 and 121, 122 and 123, 124 and 125, 126 and 127, 128 and 129 or 130 and 131, which correspond to a part of katG gene sequence that is responsible for resistance to isoniazid;
a pair of oligonucleotides having the nucleotide sequences shown as SEQ ID NOS: 132 and 133, which correspond to a part of embB gene sequence that is responsible for resistance to ethambutol, or any combinations thereof.

39. The method according to claim 35, which further comprises steps of:
performing primary nucleic acid amplification by using primary primers for amplifying a region which contains and is larger than the region of the tubercle bacilli DNA amplified by using the labeled primer; and
performing nucleic acid amplification by using the labeled primer and the amplified DNA as a template.

40. The method according to claim 39, wherein the sequence amplified by the primary nucleic acid amplification is a whole region or a region which contains a point mutation responsible for drug resistance of one of rpoB gene, rrs gene, rpsL gene, inhA gene, katG gene and embB gene, and the region which contains a point mutation is amplified more specifically by the nucleic acid amplification using the labeled primer.

41. Primary primers used for the method according to claim 40, which comprises:
a pair of oligonucleotides having the nucleotide sequences shown as SEQ ID NOS: 134 and 135 for amplifying the rpoB gene;
a pair of oligonucleotides having the nucleotide sequences shown as SEQ ID NOS: 136 and 137 for amplifying the rrs gene;
a pair of oligonucleotides having the nucleotide sequences shown as SEQ ID NOS: 138 and 139 for amplifying the rpsL gene;
a pair of oligonucleotides having the nucleotide sequences shown as SEQ ID NOS: 140 and 141 for amplifying the ihhA gene;
a pair of oligonucleotides having the nucleotide sequences shown as SEQ ID NOS: 142 and 143 for amplifying the katG gene;
a pair of oligonucleotides having the nucleotide sequences shown as SEQ ID NOS: 144 and 145 for amplifying the embB gene, or any combination thereof.

42. A method for differentiating drug resistance of tubercle bacilli, wherein a nucleic acid probe obtained by the method according to any one of claims 35, 39 and 40 is hybridized with an oligonucleotide immobilized on a substrate contained in the kit according to any one of claims 1-6 and 29-34 to identify an oligonucleotide derived from a gene responsible for drug resistance of a mutant type tubercle bacilli having a nucleotide sequence which is completely identical to the nucleic acid probe.

43. The method according to claim 35, wherein an oligonucleotide pair obtained from a gene region which is commonly contained in a wild-type tubercle bacilli susceptible to a drug and a mutant type tubercle bacilli resistant to a drug but is not genetically conserved among *Mycobacterial* species is used as the primers.

44. The method according to claim 43, wherein the pair of oligonucleotides has the nucleotide sequence shown as SEQ ID NO: 102 derived from the rrs gene.

45. The method according to claim 43, wherein, prior to the nucleic acid amplification using the pair of oligonucleotides having the nucleotide sequence shown as SEQ ID NOS: 104 and 105, the primary nucleic acid amplification is carried out by using the pair of oligonucleotides having the nucleotide sequences shown as SEQ ID NOS: 136 and 137 for amplifying the rrs gene.

46. A method for determining infection with tubercle bacilli, which comprises steps of:
hybridizing a nucleic acid probe obtained by the method according to claim 43 or 45 with an oligonucleotide immobilized on a substrate contained in the kit according to claim 32; and
comparing association property of the nucleic acid probe with respect to the positive control and the negative control to determine infection with tubercle bacilli.

47. A method for simultaneously performing differentiation of drug resistance of tubercle bacilli and determination of infection with tubercle bacilli, which comprises a step of simultaneously hybridizing the nucleic acid probe according to any one of claims 35, 39 and 40 and the nucleic acid probe obtained by the method according to any one of claims 43-45 on a substrate using a kit according to claim 1 comprising a combination of oligonucleotides of (a) and (b) and a combination of oligonucleotides of (c) and (d) mentioned below:
(a) an oligonucleotide which is derived from a mutant type gene having a point mutation responsible for the drug resistance contained in a mutant type tubercle bacilli which is resistant to any one of drugs selected from rifampicin, streptomycin, kanamycin, isoniazid and ethambutol, and contains
any one of the nucleotide sequences shown as SEQ ID NOS: 1-66; or
a nucleotide sequence which is a nucleotide sequence obtained by extending or shortening any one of the nucleotide sequences at the 5' end and/or the 3' end with or for a gene sequence of a wild-type tubercle bacilli of which position on the tubercle bacilli genome corresponds to each of the nucleotide sequences, and designed so as not to substitute the mutation contained in each of the original nucleotide sequences and to have a length of 12-24 nucleotides;
(b) an oligonucleotide which is a wild-type gene of a wild-type tubercle bacilli which is susceptible to rifampicin, streptomycin, kanamycin, isoniazid and ethambutol, and contains
any one of the nucleotide sequences shown as SEQ ID NOS: 67-101; or
a nucleotide sequence which is obtained from a gene sequence of which position on the tubercle bacilli genome corresponds to each of the nucleotide sequences of (a), and designed so as to have a length of 12-24 nucleotides;
(c) an oligonucleotide obtained from a gene region which is commonly contained in a wild-type tubercle bacilli susceptible to a drug and a mutant type tubercle bacilli resistant to a drug but is not genetically conserved among *Mycobacterial* species;
(d) an oligonucleotide obtained by substituting one or more nucleotides of a nucleotide sequence of the oligonucleotide of (c) within a range of from one nucleotide to less than 20% of the total number of nucleotides of the nucleotide sequence.

48. The kit according to claim 47, wherein the oligonucleotide of (c) contains the nucleotide sequence shown as SEQ ID NO: 102 and is derived from the rrs gene.

49. A kit for identifying infection with a *Mycobacteria* by hybridization with a nucleic acid derived from a bacterium to be tested, which comprise or use a substrate on which immobilized through a covalent bond is an oligonucleotide synthesized based on nucleotide sequences derived from genes on genome of bacteria belonging to tubercle bacilli and atypical acid-fast bacilli.

50. The kit according to claim 49, wherein a compound containing a carbodiimide group or an isocyanate group is coated on a surface, and the covalent bond is formed by a reaction between the carbodiimide group or the isocyanate group and the oligonucleotide or an amino group of a linker added to a terminal of the oligonucleotide.

51. The kit according to claim 49, wherein the oligonucleotide is immobilized on an area of the substrate surface having a diameter of 10-1000 µm.

52. The kit according to claim 49, wherein the bacterium belonging to atypical acid-fast bacilli contain at least one of:
*Mycobacterium xenopi;*
*Mycobacterium ulcerans;*
*Mycobacterium szulgai;*
*Mycobacterium simiae;*
*Mycobacterium shimoidei;*
*Mycobacterium scrofulaceum;*
*Mycobacterium nonchromogenicum;*
*Mycobacterium marinum;*
*Mycobacterium malmoense;*
*Mycobacterium intracellurare;*
*Mycobacterium gordonae;*
*Mycobacterium fortuitum;*
*Mycobacterium chelonae;*
*Mycobacterium avium* complex;
*Mycobacterium kansasii.*

53. The kit according to claim 49, wherein the gene on the genome is a 16S ribosome gene.

54. The kit according to claim 49, wherein the oligonucleotide is synthesized based on a nucleotide sequence present in the 16S ribosome gene which is specific for each of tubercle bacilli and bacteria belonging to atypical acid-fast bacilli.

55. The kit according to claim 49, wherein the oligonucleotide as a positive control contains the nucleotide sequence mentioned in claim 54, the oligonucleotide as a negative sequence consists of an oligonucleotide which has a nucleotide sequence corresponding to the positive control comprising substitution of a part of the nucleotide sequence of the positive control so that sequence-specific hybridization should not be caused, and the identification is attained by comparison of results obtained by hybridization with a nucleic acid derived from the bacterium to be tested.

56. The kit according to claim 55, wherein the positive control sequence and the negative control sequence are:
SEQ ID NO: 160 and SEQ ID NO: 161 for tubercle bacilli;
SEQ ID NO: 162 and SEQ ID NO: 165 for *Mycobacterium avium;*
SEQ ID NO: 164 and SEQ ID NO: 163 for *Mycobacterium kansasii;*
SEQ ID NO: 206 and SEQ ID NO: 219 for *Mycobacterium xenopi;*
SEQ ID NO: 207 and SEQ ID NO: 220 for *Mycobacterium ulcerans;*
SEQ ID NO: 208 and SEQ ID NO: 221 for *Mycobacterium szulgai;*
SEQ ID NO: 209 and SEQ ID NO: 222 for *Mycobacterium simiae;*
SEQ ID NO: 210 and SEQ ID NO: 223 for *Mycobacterium shimoidei;*
SEQ ID NO: 211 and SEQ ID NO: 224 for *Mycobacterium scrofulaceum;*
SEQ ID NO: 212 and SEQ ID NO: 225 for *Mycobacterium nonchromogenicum;*
SEQ ID NO: 213 and SEQ ID NO: 226 for *Mycobacterium marinum;*
SEQ ID NO: 214 and SEQ ID NO: 227 for *Mycobacterium malmoense;*
SEQ ID NO: 215 and SEQ ID NO: 228 for *Mycobacterium intracellurare;*
SEQ ID NO: 216 and SEQ ID NO: 229 for *Mycobacterium gordonae;*
SEQ ID NO: 217 and SEQ ID NO: 230 for *Mycobacterium fortuitum;* and
SEQ ID NO: 218 and SEQ ID NO: 231 for *Mycobacterium chelonae.*

57. A kit for simultaneously performing differentiation of drug resistance of tubercle bacilli and identification of infection with *Mycobacteria* including tubercle bacilli and atypical acid-fast bacilli by hybridization with a nucleic acid derived from a bacterium to be examined, which comprise or use a substrate on which immobilized through a covalent bond is an oligonucleotide synthesized based on nucleotide sequences derived from genes on genome of bacteria belonging to tubercle bacilli and atypical acid-fast bacilli.

58. The kit according to any one of claims 1, 49 and 57, wherein the nucleic acid derived from the bacterium to be examined is RNA.

59. The kit according to any one of claims 1, 49 and 57, wherein the synthetic oligonucleotide is RNA or PNA (peptide nucleic acid).

60. The kit according to any one of claims 1 to 6, 29 to 34, 49 and 57, wherein one or more nucleoside which constitutes the oligonucloside is replaced by inosine or nucleic acid which does not match with any of nuclosides to eliminate a three-dimensional structure, when the oligonucleotide has a three-dimensional structure such as a hair-pin structure or a loop structure which blocks hybridization with a nucleic acid to be tested.

61. A kit according to claim 2, the reaction of carbodiimide group or an isocyanate group on the substrate with the oligonucleotide is performed under UV irradiation.

62. A method for designing an oligonucleotide comprising a nucleotide sequence, wherein an oligonucleotide derived from a mutant gene having a point mutation, deletion mutation or insertion mutation responsible for drug resistance of drug resistant tubercle bacilli and have a length of 12-24 nucleotides containing the mutation is designed by using a database which contains genes on tubercle bacilli genome, and the oligonucleotide is extended or shortened at the 5' end and/or the 3' end with or for a gene sequence of a wild-type tubercle bacilli of which position on the tubercle bacilli genome corresponds to the nucleotide sequence to obtain the nucleotide sequence.


SEQUENCE LISTING

<110> System research Inc.
      Nisshinbo Industry Inc.

<120> Kit for Diagnosis of Tubercle Bacilli

<130> OP972

<140>
<141> 2000-08-

<150> JP 11-220357
<151> 1999-08-03

<160> 231

<170> PatentIn Ver. 2.0

<210> 1
<211> 17
<212> DNA
<213> Mycobacterium tuberculosis

<220>
<223> capture

<400> 1
gctgagcaaa ttcatgg                                                17

<210> 2
<211> 17
<212> DNA
<213> Mycobacterium tuberculosis

<220>
<223> capture

<400> 2
gctgagcgaa ttcatgg                                                    17

<210> 3
<211> 17
<212> DNA
<213> Mycobacterium tuberculosis

<220>
<223> capture

<400> 3
gctgagctaa ttcatgg                                                    17

<210> 4
<211> 17
<212> DNA
<213> Mycobacterium tuberculosis

<220>
<223> capture

<400> 4
gctgagccca ttcatgg                                                    17

<210> 5
<211> 19
<212> DNA
<213> Mycobacterium tuberculosis

<400> 5
aattcatggt ccagaacaa                                                  19

<210> 6
<211> 19
<212> DNA
<213> Mycobacterium tuberculosis

<220>
<223> capture

<400> 6
aattcatgga acagaacaa                                                    19

<210> 7
<211> 17
<212> DNA
<213> Mycobacterium tuberculosis

<400> 7
gaacaactcg ctgtcgg                                                      17

<210> 8
<211> 15
<212> DNA
<213> Mycobacterium tuberculosis

<220>
<223> capture

<400> 8
ccgctgttgg ggttg                                                        15

<210> 9
<211> 17
<212> DNA
<213> Mycobacterium tuberculosis

<220>
<223> capture

<400> 9
ggttgacgcc ccagcgc                                                      17

<210> 10
<211> 17
<212> DNA
<213> Mycobacterium tuberculosis

<220>
<223> capture

<400> 10
ggttgaccaa caagcgc                                                    17

<210> 11
<211> 17
<212> DNA
<213> Mycobacterium tuberculosis

<220>
<223> capture

<400> 11
ggttgaccga caagcgc                                                    17

<210> 12
<211> 17
<212> DNA
<213> Mycobacterium tuberculosis

<220>
<223> capture

<400> 12
ggttgaccta caagcgc                                                    17

<210> 13
<211> 17
<212> DNA
<213> Mycobacterium tuberculosis

<220>
<223> capture

<400> 13
ggttgacccc caagcgc                                                    17

<210> 14
<211> 17
<212> DNA
<213> Mycobacterium tuberculosis

<220>
<223> capture

<400> 14
ggttgacccg caagcgc                                                    17

<210> 15
<211> 17
<212> DNA
<213> Mycobacterium tuberculosis

<220>
<223> capture

<400> 15
ggttgaccca aaagcgc                                                    17

<210> 16
<211> 17
<212> DNA
<213> Mycobacterium tuberculosis

<220>
<223> capture

<400> 16
ggttgaccca gaagcgc                                                    17

<210> 17
<211> 15
<212> DNA
<213> Mycobacterium tuberculosis

<220>
<223> capture

<400> 17
cgactgtggg cgctg                                                      15

<210> 18

<211> 15
<212> DNA
<213> Mycobacterium tuberculosis

<220>
<223> capture

<400> 18
cgactgttgg cgctg                                                    15

<210> 19
<211> 14
<212> DNA
<213> Mycobacterium tuberculosis

<220>
<223> capture

<400> 19
tcggcgccgg ggcc                                                     14

<210> 20
<211> 16
<212> DNA
<213> Mycobacterium tuberculosis

<220>
<223> capture

<400> 20
gaagaagtac cggcca                                                   16

<210> 21
<211> 15
<212> DNA
<213> Mycobacterium tuberculosis

<220>
<223> capture

<400> 21

ccagtagccg cggta                                                    15

<210> 22
<211> 15
<212> DNA
<213> Mycobacterium tuberculosis

<220>
<223> capture

<400> 22
ccagtagccg cggta                                                    15

<210> 23
<211> 16
<212> DNA
<213> Mycobacterium tuberculosis

<220>
<223> capture

<400> 23
ccagctgccc gcggta                                                   16

<210> 24
<211> 15
<212> DNA
<213> Mycobacterium tuberculosis

<220>
<223> capture

<400> 24
ccagcagctg cggta                                                    15

<210> 25
<211> 19
<212> DNA
<213> Mycobacterium tuberculosis

<220>

<223> capture

<400> 25
ggctaaaact aaaaggaat                                                    19

<210> 26
<211> 19
<212> DNA
<213> Mycobacterium tuberculosis

<220>
<223> capture

<400> 26
ggctaaaact aaaaggaat                                                    19

<210> 27
<211> 19
<212> DNA
<213> Mycobacterium tuberculosis

<220>
<223> capture

<400> 27
ggctaaaact cgaaggaat                                                    19

<210> 28
<211> 15
<212> DNA
<213> Mycobacterium tuberculosis

<220>
<223> capture

<400> 28
ccgcctaggg agtac                                                        15

<210> 29
<211> 16
<212> DNA

<213> Mycobacterium tuberculosis

<220>
<223> capture

<400> 29
cactccgagg aagccg                                                    16

<210> 30
<211> 16
<212> DNA
<213> Mycobacterium tuberculosis

<220>
<223> capture

<400> 30
cactccgacg aagccg                                                    16

<210> 31
<211> 16
<212> DNA
<213> Mycobacterium tuberculosis

<220>
<223> capture

<400> 31
ggtgcaggac ctgcct                                                    16

<210> 32
<211> 16
<212> DNA
<213> Mycobacterium tuberculosis

<400> 32
ggtgagggac ctgcct                                                    16

<210> 33
<211> 16
<212> DNA

<213> Mycobacterium tuberculosis

<220>
<223> capture

<400> 33
ggtgatggac ctgcct 16

<210> 34
<211> 16
<212> DNA
<213> Mycobacterium tuberculosis

<220>
<223> capture

<400> 34
gcccgtcgcg tcatga 16

<210> 35
<211> 16
<212> DNA
<213> Mycobacterium tuberculosis

<400> 35
gcccgtcaag tcatga 16

<210> 36
<211> 16
<212> DNA
<213> Mycobacterium tuberculosis

<220>
<223> capture

<220>
<223> capture

<400> 36
gcccgtcatg tcatga 16

<210> 37
<211> 17
<212> DNA
<213> Mycobacterium tuberculosis

<220>
<223> capture

<400> 37
gattgggact aagtcgt                                                    17

<210> 38
<211> 17
<212> DNA
<213> Mycobacterium tuberculosis

<220>
<223> capture

<400> 38
gcggcgaggc gataggt                                                    17

<210> 39
<211> 17
<212> DNA
<213> Mycobacterium tuberculosis

<220>
<223> capture

<400> 39
gcggcgagat gataggt                                                    17

<210> 40
<211> 17
<212> DNA
<213> Mycobacterium tuberculosis

<220>
<223> capture

<400> 40
acgataggat gtcgggg                                                    17

<210> 41
<211> 17
<212> DNA
<213> Mycobacterium tuberculosis

<220>
<223> capture

<400> 41
acgatagggt gtcgggg                                                    17

<210> 42
<211> 17
<212> DNA
<213> Mycobacterium tuberculosis

<220>
<223> capture

<400> 42
gcccgccgcc tacggcc                                                    17

<210> 43
<211> 17
<212> DNA
<213> Mycobacterium tuberculosis

<220>
<223> capture

<400> 43
ggcgtgggag gctgccg                                                    17

<210> 44
<211> 17
<212> DNA
<213> Mycobacterium tuberculosis

<220>
<223> capture

<400> 44
ggcgccggcg gcggcat                                                    17

<210> 45
<211> 17
<212> DNA
<213> Mycobacterium tuberculosis

<220>
<223> capture

<400> 45
gcccgaccac gccagct                                                    17

<210> 46
<211> 17
<212> DNA
<213> Mycobacterium tuberculosis

<220>
<223> capture

<400> 46
gcccgacagc gccagct                                                    17

<210> 47
<211> 17
<212> DNA
<213> Mycobacterium tuberculosis

<220>
<223> capture

<400> 47
caacgccaac ttggaca                                                    17

<210> 48

<211> 17
<212> DNA
<213> Mycobacterium tuberculosis

<220>
<223> capture

<400> 48
ccagcttggc caaggcg                                                    17

<210> 49
<211> 17
<212> DNA
<213> Mycobacterium tuberculosis

<220>
<223> capture

<400> 49
gaagctctta tgggcgg                                                    17

<210> 50
<211> 17
<212> DNA
<213> Mycobacterium tuberculosis

<220>
<223> capture

<400> 50
gcaactgcac gctggaa                                                    17

<210> 51
<211> 17
<212> DNA
<213> Mycobacterium tuberculosis

<220>
<223> capture

<400> 51

cttcaagaag ttcgggt 17

<210> 52
<211> 17
<212> DNA
<213> Mycobacterium tuberculosis

<220>
<223> capture

<400> 52
tcggtaaggc ccatggc 17

<210> 53
<211> 17
<212> DNA
<213> Mycobacterium tuberculosis

<220>
<223> capture

<400> 53
gatcaccaac ggcatcg 17

<210> 54
<211> 17
<212> DNA
<213> Mycobacterium tuberculosis

<220>
<223> capture

<400> 54
gatcaccacc ggcatcg 17

<210> 55
<211> 17
<212> DNA
<213> Mycobacterium tuberculosis

<220>

<223> capture

<400> 55
gatcaccaca ggcatcg                                                    17

<210> 56
<211> 17
<212> DNA
<213> Mycobacterium tuberculosis

<220>
<223> capture

<400> 56
cgacgaaagg ggacaac                                                    17

<210> 57
<211> 17
<212> DNA
<213> Mycobacterium tuberculosis

<220>
<223> capture

<400> 57
ccagatcctg gcatcgg                                                    17

<210> 58
<211> 17
<212> DNA
<213> Mycobacterium tuberculosis

<220>
<223> capture

<400> 58
accgcatgag cggcggc                                                    17

<210> 59
<211> 17
<212> DNA

<213> Mycobacterium tuberculosis

<220>
<223> capture

<400> 59
ggtgccctcc accccgg                                                    17

<210> 60
<211> 17
<212> DNA
<213> Mycobacterium tuberculosis

<220>
<223> capture

<400> 60
ggcagatgac ttccgaa                                                    17

<210> 61
<211> 17
<212> DNA
<213> Mycobacterium tuberculosis

<220>
<223> capture

<400> 61
   cctgggcgtg gcccgag                                                   17

<210> 62
<211> 17
<212> DNA
<213> Mycobacterium tuberculosis

<220>
<223> capture

<400> 62
   cctgggcctg gcccgag                                                   17

<210> 63
<211> 17
<212> DNA
<213> Mycobacterium tuberculosis

<220>
<223> capture

<400> 63
cctgggcata gcccgag                                                    17

<210> 64
<211> 17
<212> DNA
<213> Mycobacterium tuberculosis

<220>
<223> capture

<400> 64
cctgggcatt gcccgag                                                    17

<210> 65
<211> 17
<212> DNA
<213> Mycobacterium tuberculosis

<220>
<223> capture

<400> 65
cctgggcatc gcccgag                                                    17

<210> 66
<211> 17
<212> DNA
<213> Mycobacterium tuberculosis

<220>
<223> capture

<400> 66
aggatcccgt cggctgg                                                    17

<210> 67
<211> 17
<212> DNA
<213> Mycobacterium tuberculosis

<220>
<223> capture

<400> 67
gctgagccaa ttcatgg                                                    17

<210> 68
<211> 19
<212> DNA
<213> Mycobacterium tuberculosis

<220>
<223> capture

<400> 68
aattcatgga ccagaacaa                                                  19

<210> 69
<211> 17
<212> DNA
<213> Mycobacterium tuberculosis

<220>
<223> capture

<400> 69
gaacaacccg ctgtcgg                                                    17

<210> 70
<211> 15
<212> DNA
<213> Mycobacterium tuberculosis

<220>
<223> capture

<400> 70
ccgctgtcgg ggttg                                                        15

<210> 71
<211> 17
<212> DNA
<213> Mycobacterium tuberculosis

<220>
<223> capture

<400> 71
ggttgaccca caagcgc                                                      17

<210> 72
<211> 15
<212> DNA
<213> Mycobacterium tuberculosis

<220>
<223> capture

<400> 72
cgactgtcgg cgctg                                                        15

<210> 73
<211> 14
<212> DNA
<213> Mycobacterium tuberculosis

<220>
<223> capture

<400> 73
tcggcgctgg ggcc                                                         14

<210> 74
<211> 16

<212> DNA
<213> Mycobacterium tuberculosis

<220>
<223> capture

<400> 74
gaagaagcac cggcca                                                    16

<210> 75
<211> 15
<212> DNA
<213> Mycobacterium tuberculosis

<220>
<223> capture

<400> 75
ccagcagccg cggta                                                     15

<210> 76
<211> 15
<212> DNA
<213> Mycobacterium tuberculosis

<220>
<223> capture

<400> 76
ccgcctgggg agtac                                                     15

<210> 77
<211> 19
<212> DNA
<213> Mycobacterium tuberculosis

<220>
<223> capture

<400> 77
ggctaaaact caaaggaat                                                 19

<210> 78
<211> 16
<212> DNA
<213> Mycobacterium tuberculosis

<220>
<223> capture

<400> 78
cactccgaag aagccg                                                    16

<210> 79
<211> 16
<212> DNA
<213> Mycobacterium tuberculosis

<220>
<223> capture

<400> 79
ggtgaaggac ctgcct                                                    16

<210> 80
<211> 16
<212> DNA
<213> Mycobacterium tuberculosis

<220>
<223> capture

<400> 80
gcccgtcacg tcatga                                                    16

<210> 81
<211> 17
<212> DNA
<213> Mycobacterium tuberculosis

<220>
<223> capture

<400> 81
gattgggacg aagtcgt                                                    17

<210> 82
<211> 17
<212> DNA
<213> Mycobacterium tuberculosis

<220>
<223> capture

<400> 82
gcggcgagac gataggt                                                    17

<210> 83
<211> 17
<212> DNA
<213> Mycobacterium tuberculosis

<220>
<223> capture

<400> 83
acgataggtt gtcgggg                                                    17

<210> 84
<211> 17
<212> DNA
<213> Mycobacterium tuberculosis

<220>
<223> capture

<400> 84
gcccgccgac tacggcc                                                    17

<210> 85
<211> 17
<212> DNA
<213> Mycobacterium tuberculosis

<220>
<223> capture

<400> 85
ggcgtggcac gctgccg                                                    17

<210> 86
<211> 17
<212> DNA
<213> Mycobacterium tuberculosis

<220>
<223> capture

<400> 86
ggcgccgggg gcggcat                                                    17

<210> 87
<211> 17
<212> DNA
<213> Mycobacterium tuberculosis

<220>
<223> capture

<400> 87
gcccgacaac gccagct                                                    17

<210> 88
<211> 17
<212> DNA
<213> Mycobacterium tuberculosis

<220>
<223> capture

<400> 88
caacgccagc ttggaca                                                    17

<210> 89

<211> 17
<212> DNA
<213> Mycobacterium tuberculosis

<220>
<223> capture

<400> 89
ccagcttgga caaggcg                                                    17

<210> 90
<211> 17
<212> DNA
<213> Mycobacterium tuberculosis

<220>
<223> capture

<400> 90
gaagctctca tgggcgg                                                    17

<210> 91
<211> 17
<212> DNA
<213> Mycobacterium tuberculosis

<220>
<223> capture

<400> 91
gcaactgcgc gctggaa                                                    17

<210> 92
<211> 17
<212> DNA
<213> Mycobacterium tuberculosis

<220>
<223> capture

<400> 92

cttcaagacg ttcgggt 17

<210> 93
<211> 17
<212> DNA
<213> Mycobacterium tuberculosis

<220>
<223> capture

<400> 93
tcggtaagac ccatggc 17

<210> 94
<211> 17
<212> DNA
<213> Mycobacterium tuberculosis

<220>
<223> capture

<400> 94
gatcaccagc ggcatcg 17

<210> 95
<211> 17
<212> DNA
<213> Mycobacterium tuberculosis

<220>
<223> capture

<400> 95
cgacgaaatg ggacaac 17

<210> 96
<211> 17
<212> DNA
<213> Mycobacterium tuberculosis

<220>

<223> capture

<400> 96
ccagatccgg gcatcgg                                                    17

<210> 97
<211> 17
<212> DNA
<213> Mycobacterium tuberculosis

<220>
<223> capture

<400> 97
accgcatggg cggcggc                                                    17

<210> 98
<211> 17
<212> DNA
<213> Mycobacterium tuberculosis

<220>
<223> capture

<400> 98
ggtgcccttc accccgg                                                    17

<210> 99
<211> 17
<212> DNA
<213> Mycobacterium tuberculosis

<220>
<223> capture

<400> 99
ggcagatggc ttccgaa                                                    17

<210> 100
<211> 17
<212> DNA

<213> Mycobacterium tuberculosis

<220>
<223> capture

<400> 100
cctgggcatg gcccgag                                                    17

<210> 101
<211> 17
<212> DNA
<213> Mycobacterium tuberculosis

<220>
<223> capture

<400> 101
aggatccctt cggctgg                                                    17

<210> 102
<211> 17
<212> DNA
<213> Mycobacterium tuberculosis

<220>
<223> capture

<400> 102
cacgggatgc atgtctt                                                    17

<210> 103
<211> 17
<212> DNA
<213> Mycobacterium tuberculosis

<220>
<223> capture

<400> 103
cacggggcgc atgtctt                                                    17

<210> 104
<211> 20
<212> DNA
<213> Mycobacterium tuberculosis

<220>
<223> primer: amplify  M. tuberculosis rrs gene

<400> 104
tgggaaactg ggtctaatac                                                    20

<210> 105
<211> 20
<212> DNA
<213> Mycobacterium tuberculosis

<220>
<223> primer: amplify rrs gene

<220>
<223> biotin at 5' terminus

<400> 105
gcgggctcat cccacaccgc                                                    20

<210> 106
<211> 17
<212> DNA
<213> Mycobacterium tuberculosis

<220>
<223> primer: amplify rpoB gene

<400> 106
gccgcgatca aggagtt                                                       17

<210> 107
<211> 17
<212> DNA
<213> Mycobacterium tuberculosis

<220>
<223> primer:amplify rpoB gene

<220>
<223> biotin at 5' terminus

<400> 107
cacgtgacag accgccg                                                    17

<210> 108
<211> 20
<212> DNA
<213> Mycobacterium tuberculosis

<220>
<223> primer:amplify rrs 530 region

<400> 108
tctctcggat tgacggtagg                                                 20

<210> 109
<211> 20
<212> DNA
<213> Mycobacterium tuberculosis

<220>
<223> primer:amplify rrs530 region

<220>
<223> biotin at 5' terminus

<400> 109
cctacgagct ctttacgccc                                                 20

<210> 110
<211> 20
<212> DNA
<213> Mycobacterium tuberculosis

<220>
<223> primer:amplify rrs 915 region

<400> 110
gatccgtgcc gtagctaacg                                                    20

<210> 111
<211> 20
<212> DNA
<213> Mycobacterium tuberculosis

<220>
<223> primer:amplify rrs 915 region

<220>
<223> biotin at 5' terminus

<400> 111
cgcttgtgcg ggcccccgtc                                                    20

<210> 112
<211> 20
<212> DNA
<213> Mycobacterium tuberculosis

<220>
<223> primer:amplify rpsL gene

<400> 112
gcacccgcgt gtacaccacc                                                    20

<210> 113
<211> 20
<212> DNA
<213> Mycobacterium tuberculosis

<220>
<223> primer:amplify rpsL gene

<220>
<223> biotin at 5' terminus

<400> 113

ttgtagcgca caccaggcag 20

<210> 114
<211> 20
<212> DNA
<213> Mycobacterium tuberculosis

<220>
<223> primer:amplify rrs 1400 region

<400> 114
tcccgggcct tgtacacacc 20

<210> 115
<211> 20
<212> DNA
<213> Mycobacterium tuberculosis

<220>
<223> primer: amplify rrs 1400 region

<220>
<223> biotin at 5' terminus

<400> 115
cttccggtac ggctaccttg 20

<210> 116
<211> 20
<212> DNA
<213> Mycobacterium tuberculosis

<220>
<223> primer: amplify inhA gene

<400> 116
gaaagttccc gccggaaatc 20

<210> 117
<211> 20
<212> DNA

<213> Mycobacterium tuberculosis

<220>
<223> primer:amplify inhA gene

<220>
<223> biotin at 5' terminus

<400> 117
actgaacggg atacgaatgg                                                    20

<210> 118
<211> 20
<212> DNA
<213> Mycobacterium tuberculosis

<220>
<223> primer:amplify katG gene

<400> 118
ggtcgcgacc atcgacgttg                                                    20

<210> 119
<211> 20
<212> DNA
<213> Mycobacterium tuberculosis

<220>
<223> primer:amplify katG gene

<220>
<223> biotin at 5' terminus

<400> 119
tcgtggatgc ggtaggtgcc                                                    20

<210> 120
<211> 20
<212> DNA
<213> Mycobacterium tuberculosis

<220>
<223> primer: amplify katG gene

<400> 120
ggcacctacc gcatccacga                                                    20

<210> 121
<211> 20
<212> DNA
<213> Mycobacterium tuberculosis

<220>
<223> primer: amplify katG gene

<220>
<223> biotin at 5' terminus

<400> 121
tgagagcttc ttgccgtact                                                    20

<210> 122
<211> 20
<212> DNA
<213> Mycobacterium tuberculosis

<220>
<223> primer: amplify katG gene

<400> 122
gctgtggccg gtcaagaaga                                                    20

<210> 123
<211> 20
<212> DNA
<213> Mycobacterium tuberculosis

<220>
<223> primer:amplify katG gene

<220>
<223> biotin at 5' terminus

<400> 123
tagacctcat cgggctccca                                                    20

<210> 124
<211> 20
<212> DNA
<213> Mycobacterium tuberculosis

<220>
<223> primer: amplify katG gene

<400> 124
cattcgcgag acgtttcggc                                                    20

<210> 125
<211> 20
<212> DNA
<213> Mycobacterium tuberculosis

<220>
<223> primer: amplify katG gene

<220>
<223> biotin at 5' terminus

<400> 125
cccatctgct ccagcggagc                                                    20

<210> 126
<211> 20
<212> DNA
<213> Mycobacterium tuberculosis

<220>
<223> primer: amplify katG gene

<400> 126
aagagctcgt atggcaccgg                                                    20

<210> 127

<211> 20
<212> DNA
<213> Mycobacterium tuberculosis

<220>
<223> primer: amplify katG gene

<220>
<223> biotin at 5' terminus

<400> 127
agcgccagca gggctcttcg                                                    20

<210> 128
<211> 20
<212> DNA
<213> Mycobacterium tuberculosis

<220>
<223> primer: amplify katG gene

<400> 128
ggcgaagccg agattgccag                                                    20

<210> 129
<211> 20
<212> DNA
<213> Mycobacterium tuberculosis

<220>
<223> primer:amplify katG gene

<220>
<223> biotin at 5' terminus

<400> 129
ctgcaggcgg atgcgaccac                                                    20

<210> 130
<211> 20
<212> DNA

<213> Mycobacterium tuberculosis

<220>
<223> primer: amplify katG gene

<400> 130
aagcagcaaa ggcggctggc                                                    20

<210> 131
<211> 20
<212> DNA
<213> Mycobacterium tuberculosis

<220>
<223> primer: amplify katG gene

<220>
<223> biotin at 5' terminus

<400> 131
acgggttgcc ctttccgagg                                                    20

<210> 132
<211> 20
<212> DNA
<213> Mycobacterium tuberculosis

<220>
<223> primer: amplify embB gene

<400> 132
gcgcgaattc gtcggacgac                                                    20

<210> 133
<211> 20
<212> DNA
<213> Mycobacterium tuberculosis

<220>
<223> primer: amplify embB gene

<220>
<223> biotin at 5' terminus

<400> 133
tgacatgggt catcagcgcc                                                    20

<210> 134
<211> 20
<212> DNA
<213> Mycobacterium tuberculosis

<220>
<223> primer:amplify rpoB gene

<400> 134
cgttgatcaa catccggccg                                                    20

<210> 135
<211> 20
<212> DNA
<213> Mycobacterium tuberculosis

<220>
<223> primer: amplify rpoB gene

<400> 135
tgcacgtcgc ggacctccag                                                    20

<210> 136
<211> 20
<212> DNA
<213> Mycobacterium tuberculosis

<220>
<223> primer:amplify rrs gene

<400> 136
agagtttgat cctggctcag                                                    20

<210> 137
<211> 20

<212> DNA
<213> Mycobacterium tuberculosis

<220>
<223> primer: amplify rrs gene

<400> 137
tacggctacc ttgttacgac                                                    20

<210> 138
<211> 20
<212> DNA
<213> Mycobacterium tuberculosis

<220>
<223> primer: amplify rpsL gene

<400> 138
gcggctctga agggcagccc                                                    20

<210> 139
<211> 20
<212> DNA
<213> Mycobacterium tuberculosis

<220>
<223> primer: amplify rpsL gene

<400> 139
gtttgcggtt cttgacaccc                                                    20

<210> 140
<211> 20
<212> DNA
<213> Mycobacterium tuberculosis

<220>
<223> primer: ampify inhA gene

<400> 140
caccgacaaa cgtcacgagc                                                    20

<210> 141
<211> 20
<212> DNA
<213> Mycobacterium tuberculosis

<220>
<223> primer:amplify inhA gene

<400> 141
gccgctgtgc gatcgccagc                                                    20

<210> 142
<211> 20
<212> DNA
<213> Mycobacterium tuberculosis

<220>
<223> primer: amplify katG gene

<400> 142
cgatccctgc ggcgttcgac                                                    20

<210> 143
<211> 20
<212> DNA
<213> Mycobacterium tuberculosis

<220>
<223> primer: amplify katG gene

<400> 143
ccttgtcgag cagcatgtac                                                    20

<210> 144
<211> 20
<212> DNA
<213> Mycobacterium tuberculosis

<220>
<223> primer: amplify embB gene

<400> 144
tgatattcgg cttcctgctc                                                    20

<210> 145
<211> 20
<212> DNA
<213> Mycobacterium tuberculosis

<220>
<223> primer: amplify embB gene

<400> 145
gcggccaggt ctggcaggcg                                                    20

<210> 146
<211> 124
<212> DNA
<213> Mycobacterium tuberculosis

<220>
<223> nucleotide probe: amplified from M. tuberculosis
                        rpoB gene

<400> 146
gccgcgatca aggagttctt cggcaccagc cagctgagcc aattcatgga ccagaacaac 60
ccgctgtcgg ggttgaccca caagcgccga ctgtcggcgc tggggcccgg cggtctgtca 120
cgtg                                                                     124

<210> 147
<211> 116
<212> DNA
<213> Mycobacterium tuberculosis

<220>
<223> nucleotide probe: amplified from M. tuberculosis
                        rrs gene

<400> 147
tctctcggat tgacggtagg tggagaagaa gcaccggcca actacgtgcc agcagccgcg 60
gtaatacgta gggtgcgagc gttgtccgga attactgggc gtaaagagct cgtagg      116

<210> 148
<211> 94
<212> DNA
<213> Mycobacterium tuberculosis

<220>
<223> nucleotide probe: amplified from M. tuberculosis
rrs gene

<400> 148
gatccgtgcc gtagctaacg cattaagtac cccgcctggg gagtacggcc gcaaggctaa 60
aactcaaagg aattgacggg ggcccgcaca agcg 94

<210> 149
<211> 187
<212> DNA
<213> Mycobacterium tuberculosis

<220>
<223> nucleotide probe: amplified from M. tuberculosis
rpsL gene

<400> 149
gcacccgcgt gtacaccacc actccgaaga agccgaactc ggcgcttcgg aaggttgccc 60
gcgtgaagtt gacgagtcag gtcgaggtca cggcgtacat tcccggcgag ggccacaacc 120
tgcaggagca ctcgatggtg ctggtgcgcg gcggccgggt gaaggacctg cctggtgtgc 180
gctacaa 187

<210> 150
<211> 140
<212> DNA
<213> Mycobacterium tuberculosis

<220>
<223> nucleotide probe: amplified from M. tuberculosis
rrs gene

<400> 150
tcccgggcct tgtacacacc gcccgtcacg tcatgaaagt cggtaacacc cgaagccagt 60
ggcctaaccc tcgggaggga gctgtcgaag gtgggatcgg cgattgggac gaagtcgtaa 120

caaggtagcc gtaccggaag                                                140

<210> 151
<211> 140
<212> DNA
<213> Mycobacterium tuberculosis

<220>
<223> nucleotide probe: amplified from M. tuberculosis
                        inhA gene

<400> 151
gaaagttccc gccggaaatc gcagccacgt tacgctcgtg gacataccga tttcggcccg 60
gccgcggcga gacgataggt tgtcggggtg actgccacag ccactgaagg ggccaaaccc 120
ccattcgtat cccgttcagt                                                140

<210> 152
<211> 150
<212> DNA
<213> Mycobacterium tuberculosis

<220>
<223> nucleotide probe: amplified from M. tuberculosis
                        katG gene

<400> 152
ggtcgcgacc atcgacgttg acgccctgac gcgggacatc gaggaagtga tgaccacctc 60
gcagccgtgg tggcccgccg actacggcca ctacgggccg ctgtttatcc ggatggcgtg 120
gcacgctgcc ggcacctacc gcatccacga                                     150

<210> 153
<211> 150
<212> DNA
<213> Mycobacterium tuberculosis

<220>
<223> nucleotide probe: amplified from M. tuberculosis
                        katG gene

<400> 153
ggcacctacc gcatccacga cggccgcggc ggcgccgggg gcggcatgca gcggttcgcg 60

ccgcttaaca gctggcccga caacgccagc ttggacaagg cgcgccggct gctgtggccg 120
gtcaagaaga agtacggcaa gaagctctca 150

<210> 154
<211> 150
<212> DNA
<213> Mycobacterium tuberculosis

<220>
<223> nucleotide probe: amplified from M. tuberculosis
katG gene

<400> 154
gctgtggccg gtcaagaaga agtacggcaa gaagctctca tgggcggacc tgattgtttt 60
cgccggcaac tgcgcgctgg aatcgatggg cttcaagacg ttcgggttcg gcttcggccg 120
ggtcgaccag tgggagcccg atgaggtcta 150

<210> 155
<211> 150
<212> DNA
<213> Mycobacterium tuberculosis

<220>
<223> nucleotide probe: amplified from M. tuberculosis
katG gene

<400> 155
cattcgcgag acgtttcggc gcatggccat gaacgacgtc gaaacagcgg cgctgatcgt 60
cggcggtcac actttcggta agacccatgg cgccggcccg gccgatctgg tcggccccga 120
acccgaggct gctccgctgg agcagatggg 150

<210> 156
<211> 150
<212> DNA
<213> Mycobacterium tuberculosis

<220>
<223> nucleotide probe: amplified from M. tuberculosis
katG gene

<400> 156

aagagctcgt atggcaccgg aaccggtaag gacgcgatca ccagcggcat cgaggtcgta 60
tggacgaaca ccccgacgaa atgggacaac agtttcctcg agatcctgta cggctacgag 120
tgggagctga cgaagagccc tgctggcgct 150

<210> 157
<211> 150
<212> DNA
<213> Mycobacterium tuberculosis

<220>
<223> nucleotide probe: amplified from M. tuberculosis
katG gene

<400> 157
ggcgaagccg agattgccag ccttaagagc cagatccggg catcgggatt gactgtctca 60
cagctagttt cgaccgcatg ggcggcggcg tcgtcgttcc gtggtagcga caagcgcggc 120
ggcgccaacg gtggtcgcat ccgcctgcag 150

<210> 158
<211> 150
<212> DNA
<213> Mycobacterium tuberculosis

<220>
<223> nucleotide probe: amplified from M. tuberculosis
katG gene

<400> 158
aagcagcaaa ggcggctggc cacaacatca cggtgccctt caccccgggc cgcacggatg 60
cgtcgcagga acaaaccgac gtggaatcct ttgccgtgct ggagcccaag gcagatggct 120
tccgaaacta cctcggaaag ggcaacccgt 150

<210> 159
<211> 150
<212> DNA
<213> Mycobacterium tuberculosis

<220>
<223> nucleotide probe: amplified from M. tuberculosis
embB gene

<400> 159
gcgcgaattc gtcggacgac ggctacatcc tgggcatggc ccgagtcgcc gaccacgccg 60
gctacatgtc caactatttc cgctggttcg gcagcccgga ggatcccttc ggctggtatt 120
acaacctgct ggcgctgatg acccatgtca 150

<210> 160
<211> 18
<213> Mycobacterium tuberculosis

<220>
<223> capture

<400> 160
accacgggat gcatgtct 18

<210> 161
<211> 18
<213> artificial sequence

<220>
<223> capture

<400> 161
accacgaaat gcatgtct 18

<210> 162
<211> 18
<213> Mycobacterium avium complex

<220>
<223> capture

<400> 162
taggacctca agacgcat 18

<210> 163
<211> 18
<213> artificial sequence

<220>
<223> capture

<400> 163
cacgtaacaa tctgccct                                                    18

<210> 164
<211> 18
<213> Mycobacterium kansasii

<220>
<223> capture

<400> 164
cacgtggcaa tctgccct                                                    18

<210> 165
<211> 18
<213> artificial sequence

<220>
<223> capture

<400> 165
taggaaatca agacgcat                                                    18

<210> 166
<211> 19
<213> Mycobacterium tuberculosis

<220>
<223> capture

<400> 166
agctgagcca attcatgga                                                   19

<210> 167
<211> 16
<213> Mycobacterium tuberculosis

<220>
<223> capture

<400> 167
gctgagcaaa ttcatg                                                    16

<210> 168
<211> 17
<213> Mycobacterium tuberculosis

<220>
<223> capture

<400> 168
gctgagctaa ttcatgg                                                   17

<210> 169
<211> 18
<213> Mycobacterium tuberculosis

<220>
<223> capture

<400> 169
agctgagccc attcatgg                                                  18

<210> 170
<211> 18
<213> Mycobacterium tuberculosis

<220>
<223> capture

<400> 170
aattcatggt ccagaaca                                                  18

<210> 171
<211> 17
<213> Mycobacterium tuberculosis

<220>
<223> capture

<400> 171

attcatggaa cagaaca 17

<210> 172
<211> 21
<213> Mycobacterium tuberculosis

<220>
<223> capture

<400> 172
acagaacaac ccgctgtcggg 21

<210> 173

<400> 173
000

<210> 174
<211> 15
<213> Mycobacterium tuberculosis

<220>
<223> capture

<400> 174
ccgctgttgg ggttg 15

<210> 175
<211> 18
<213> Mycobacterium tuberculosis

<220>
<223> capture

<400> 175
ggttgaccca caagcgcc 18

<210> 176
<211> 16
<213> Mycobacterium tuberculosis

<220>
<223> capture

<400> 176
gttgacgccc cagcgc 16

<210> 177
<211> 16
<213> Mycobacterium tuberculosis

<220>
<223> capture

<400> 177
ggttgaccaa caagcg                                                    16

<210> 178
<211> 16
<213> Mycobacterium tuberculosis

<220>
<223> capture

<400> 178
ggttgaccga caagcg                                                    16

<210> 179
<211> 17
<213> Mycobacterium tuberculosis

<220>
<223> capture

<400> 179
ggttgaccta caagcgc                                                   17

<210> 180
<211> 20
<213> Mycobacterium tuberculosis

<220>
<223> capture

<400> 180
gggttgaccc ccaagcgccg                                                20

<210> 181
<211> 18
<213> Mycobacterium tuberculosis

<220>
<223> capture

<400> 181
ggttgacccg caagcgcc                                                    18

<210> 182
<211> 17
<213> Mycobacterium tuberculosis

<220>
<223> capture

<400> 182
ggttgaccca aaagcgc                                                     17

<210> 183
<211> 17
<213> Mycobacterium tuberculosis

<220>
<223> capture

<400> 183
ggttgaccca gaagcgc                                                     17

<210> 184
<211> 17
<213> Mycobacterium tuberculosis

<220>
<223> capture

<400> 184
ccgactgtcg gcgctgg                                                     17

<210> 185

<211> 14
<213> Mycobacterium tuberculosis

<220>
<223> capture

<400> 185
gactgtgggc gctg                                                    14

<210> 186
<211> 14
<213> Mycobacterium tuberculosis

<220>
<223> capture

<400> 186
cgactgttgg cgct                                                    14

<210> 187
<211> 17
<213> Mycobacterium tuberculosis

<220>
<223> capture

<400> 187
tgtcggcgct ggggccc                                                 17

<210> 188
<211> 18
<213> Mycobacterium tuberculosis

<220>
<223> capture

<400> 188
agaagaagca ccggccaa                                                18

<210> 189
<211> 17

<213> Mycobacterium tuberculosis

<220>
<223> capture

<400> 189
tgccagccgc cgcggta                                                    17

<210> 190
<211> 16
<213> Mycobacterium tuberculosis

<220>
<223> capture

<400> 190
gctaaaacta aaagga                                                     16

<210> 191
<211> 17
<213> Mycobacterium tuberculosis

<220>
<223> capture

<400> 191
gctaaaactg aaaggaa                                                    17

<210> 192
<211> 15
<213> Mycobacterium tuberculosis

<220>
<223> capture

<400> 192
ggtgatggac ctgcc                                                      15

<210> 193
<211> 17
<213> Mycobacterium tuberculosis

<220>
<223> capture

<400> 193
cccgcctggg gagtacg                                                    17

<210> 194
<211> 23
<213> Mycobacterium tuberculosis

<220>
<223> capture

<400> 194
caccactccg aagaagccga act                                             23

<210> 195
<211> 15
<213> Mycobacterium tuberculosis

<220>
<223> capture

<400> 195
ggtgcaggac ctgcc                                                      15

<210> 196
<211> 15
<213> Mycobacterium tuberculosis

<220>
<223> capture

<400> 196
ggtgagggac ctgcc                                                      15

<210> 197
<211> 14
<213> Mycobacterium tuberculosis

<220>
<223> capture

<400> 197
gtgatggacc tgcc                                                    14

<210> 198
<211> 18
<213> Mycobacterium tuberculosis

<220>
<223> capture

<400> 198
cccgtcacgt catgaaag                                                18

<210> 199
<211> 18
<213> Mycobacterium tuberculosis

<220>
<223> capture

<400> 199
cccgtcgcgt catgaaag                                                18

<210> 200
<211> 18
<213> Mycobacterium tuberculosis

<220>
<223> capture

<400> 200
accgcccgtc aagtcatg                                                18

<210> 201
<211> 18
<213> Mycobacterium tuberculosis

<220>

<223> capture

<400> 201
accgcccgtc atgtcatg                                                    18

<210> 202
<211> 16
<213> Mycobacterium tuberculosis

<220>
<223> capture

<400> 202
attgggacga agtcgt                                                      16

<210> 203
<211> 16
<213> Mycobacterium tuberculosis

<220>
<223> capture

<400> 203
attgggacta agtcgt                                                      16

<210> 204
<211> 18
<213> Mycobacteriae

<220>
<223> primer amplify rrs gene

<400> 204
ctcgagtggc gaacgggt                                                    18

<210> 205
<211> 16
<213> Mycobacteriae

<220>
<223> primer amplify rrs gene

<400> 205
agctgatagg ccgcgggc                                                    16

<210> 206
<211> 18
<213> Mycobacterium xenopi

<220>
<223> capture

<400> 206
taggaccatt ctgcgcat                                                    18

<210> 207
<211> 18
<213> Mycobacterium ulcerans

<220>
<223> capture

<400> 207
accacgggat tcatgtcc                                                    18

<210> 208
<211> 18
<213> Mycobacterium szukgai

<220>
<223> capture

<400> 208
taggaccccg aggcgcat                                                    18

<210> 209
<211> 18
<213> Mycobacterium  simiae

<220>
<223> capture

<400> 209
cacgtgggta atctgccc                                                    18

<210> 210
<211> 18
<213> Mycobacterium shimoidei

<220>
<223> capture

<400> 210
accacatgtc gcatggtg                                                    18

<210> 211
<211> 18
<213> Mycobacterium scrofulaceum

<220>
<223> capture

<400> 211
cacgtgggca atctgccc                                                    18

<210> 212
<211> 18
<213> Mycobacterium nonchromogen

<220>
<223> capture

<400> 212
accgcatgct gcatggtg                                                    18

<210> 213
<211> 18
<213> Mycobacterium marinum

<220>
<223> capture

<400> 213

accacgggat tcatgtcc 18

<210> 214
<211> 18
<213> Mycobacterium malmoense

<220>
<223> capture

<400> 214
accccgaggc gcatgcct 18

<210> 215
<211> 18
<213> Mycobacterium intracellular

<220>
<223> capture

<400> 215
taggaccttt aggcgcat 18

<210> 216
<211> 18
<213> Mycobacterium gordonae

<220>
<223> capture

<400> 216
taggaccaca ggacacat 18

<210> 217
<211> 18
<213> Mycobacterium fortuitum

<220>
<223> capture

<400> 217
tatgaccgcg cacttcct 18

<210> 218
<211> 18
<213> Mycobacterium chelonae

<220>
<223> capture

<400> 218
taggaccaca cacttcat                                                    18

<210> 219
<211> 18
<213> artificial sequence

<220>
<223> capture

<400> 219
taggaccatt ctaagcat                                                    18

<210> 220
<211> 18
<213> artificial sequence

<220>
<223> capture

<400> 220
accacgaaat tcatgtcc                                                    18

<210> 221
<211> 18
<213> artificial sequence

<220>
<223> capture

<400> 221
taggaccccg aaacgcat                                                    18

<210> 222
<211> 18
<213> artificial sequence

<220>
<223> capture

<400> 222
cacgtgaata atctgccc                                                    18

<210> 223
<211> 18
<213> artificial sequence

<220>
<223> capture

<400> 223
accacatata gcatggtg                                                    18

<210> 224
<211> 18
<213> artificial sequence

<220>
<223> capture

<400> 224
cacgtgaaca atctgccc                                                    18

<210> 225
<211> 18
<213> artificial sequence

<220>
<223> capture

<400> 225
accgcatgct aaatggtg                                                    18

<210> 226

<211> 18
<213> artificial sequence

<220>
<223> capture

<400> 226
accacgaaat tcatgtcc 18

<210> 227
<211> 18
<213> artificial sequence

<220>
<223> capture

<400> 227
accccgaggc aaatgcct 18

<210> 228
<211> 18
<213> artificial sequence

<220>
<223> capture

<400> 228
taggaccttt aggcaaat 18

<210> 229
<211> 18
<213> artificial sequence

<220>
<223> capture

<400> 229
taggaccaca aaacacat 18

<210> 230
<211> 18

<213> artificial sequence

<220>
<223> capture

<400> 230
tatgaccgca aacttcct                                                    18

<210> 231
<211> 18
<213> artificial sequence

<220>
<223> capture

<400> 231
taggacaaaa cacttcat                                                    18